# EUROPEAN PATENT APPLICATION

(11) **EP 3 964 231 A1**
(43) Date of publication of application: **09.03.2022**
(21) Application number: 21175121.9
(22) Date of filing: 06.11.2014
(51) Int. Cl.: A61K 39/21, C12P 19/34, C07K 14/16

(54) **HIV-1 ENV DNA VACCINE PLUS PROTEIN BOOST**

(30) Priority: 14.11.2013 US 201361904416 P
(62) Divisional of application: 14861787.1
(71) Applicant: Inovio Pharmaceuticals, Inc., Plymouth Meeting, PA 19462 (US); The Trustees of The University of Pennsylvania, Philadelphia, PA 19104 (US)
(72) Inventor: WEINER, David B., Merion, PA 19066 (US); MUTHUMANI, Karuppiah, Cherry Hill, NJ 08002 (US); WISE, Megan, Raleigh, NC 27613 (US); YAN, Jian, Wallingford, PA 19086 (US); BRODERICK, Kate, San Diego, CA 92120 (US)
(74) Representative: Leonard, Thomas Charles

(57) **Abstract**

The present invention is directed to an effective HIV vaccine will most likely require the induction of strong T-cell responses, broadly neutralizing antibodies (bNAbs), and the elicitation of antibody-dependent cellular cytotoxicity (ADCC). Previously, we demonstrated the induction of strong HIV/SIV cellular immune responses in macaques and humans using synthetic consensus DNA immunogens delivered via adaptive electroporation (EP). However, the ability of this improved DNA approach to prime for relevant antibody responses has not been previously studied.

Here, we investigate the immunogenicity of consensus DNA constructs encoding gp140 sequences from HIV-1 subtypes A, B, C and D in a DNA prime protein boost vaccine regimen. Mice and Guinea pigs were primed with single and multi-clade DNA via EP and boosted with recombinant gp120 protein. Sera were analyzed for gp120 binding and induction of neutralizing antibody activity. Immunization with recombinant Env protein alone induced low-titer binding antibodies with limited neutralization breath. In contrast the synthetic DNA prime protein boost protocol was induced significantly higher antibody binding titers. Furthermore, sera from DNA prime-protein boost groups were able to neutralize a broader range of viruses in a panel of tier 1 clade B viruses as well as multiple tier 1 clade A and clade C viruses. Further investigation of synthetic DNA prime + adaptive EP plus protein boost appears warranted.

## Description

### CROSS-REFERENCE TO RELATED APPLIATIONS

This application claims priority to U.S. Provisional Patent Application No. 61/904,416, filed November 14, 2013, all of which is hereby incorporated by reference.

### FIELD OF THE INVENTION

The present invention relates to treating and preventing symptoms of an associated HIV infection using a priming vaccine containing a DNA encoding the antigen, and a second vaccine for boosting the response to the first vaccine using the same or different antigen than the first vaccine.

### BACKGROUND OF THE INVENTION

There is an urgent need for improved vaccination approaches against HIV that induce improved humoral and cellular immune responses. It is generally agreed upon that strong T-cell responses and breath in neutralizing antibodies will likely play a role in the development of a protective vaccine. Though DNA platforms in the past have been poor inducers of seroconversion, recent improvements in construct design, improved delivery, and improved formulations have enhanced the immune potency of this approach. We have recently reported the induction of strong HIV/SIV-specific cellular immune responses in mice, macaques and humans using consensus DNA immunogens delivered via electroporation (EP). While these studies have confirmed the induction of a potent and broad cell-mediated response, the ability of this improved DNA-EP platform to induce or prime for neutralizing antibodies (NAbs) is unknown. Due to a heightened interest in trying to improve immune responses to HIV included by DNA prime-protein boost vaccination strategies, here we studied this combination focused on increasing binding titers and neutralization capacity in vivo.

There is a need in the art to study the immunogenicity of a synthetic consensus DNA vaccine encoding gp 140 constructs derived from individual HIV-1 subtypes A, B, C and D in a DNA prime-protein boost regimen. These consensus DNA constructs can be optimized using the following plasmid-enhancement techniques: codon optimization, RNA optimization, leader sequence addition, plasmid production at high concentrations and the DNA was delivered by adaptive EP as previously described. The DNA prime can be followed by a protein boost with recombinant HIV gp120. Immune responses were measured by ELISA, B-cell ELISpot, T-cell ELISpot, and in a TZM-bl neutralization assay. The combination approach increased T cell and antibody functionality over these observed with either independent modality.

### SUMMARY OF THE INVENTION

The present invention is directed to a composition comprising (a) a first vaccine and (b) a second vaccine. The first vaccine may comprise at least one, at least two, at least three, or at least four nucleic acids, wherein each nucleic acid may encode an antigen, and wherein the at least one, at least two, at least three, or at least four nucleic acids may be selected from the group consisting of a nucleic acid encoding a HIV-1 subtype A consensus antigen, a nucleic acid encoding a HIV-1 subtype B consensus antigen, a nucleic acid encoding a HIV-1 subtype C consensus antigen, a nucleic acid encoding a HIV-1 subtype D consensus antigen, and any combination thereof. The second vaccine may comprise at least one, at least two, at least three, or at least four antigenic peptides, wherein the at least one, at least two, at least three, or at least four antigenic peptides may be selected from the group consisting of a HIV-1 subtype A consensus peptide, a HIV-1 subtype B consensus peptide, a HIV-1 subtype C consensus peptide, a HIV-1 subtype D consensus peptide, and any combination thereof.

The present invention is also directed to a method of immunizing a subject in need thereof against HIV-1. The method comprises administering a composition comprising (a) a first vaccine and (b) a second vaccine to the subject. The first vaccine may be administered independently of the second vaccine. The first vaccine may comprise at least one, at least two, at least three, or at least four nucleic acids, wherein each nucleic acid may encode an antigen, and wherein the at least one, at least two, at least three, or at least four nucleic acids may be selected from the group consisting of a nucleic acid encoding a HIV-1 subtype A consensus antigen, a nucleic acid encoding a HIV-1 subtype B consensus antigen, a nucleic acid encoding a HIV-1 subtype C consensus antigen, a nucleic acid encoding a HIV-1 subtype D consensus antigen, and any combination thereof. The second vaccine may comprise at least one, at least two, at least three, or at least four antigenic peptides, wherein the at least one, at least two, at least three, or at least four antigenic peptides may be selected from the group consisting of a HIV-1 subtype A consensus peptide, a HIV-1 subtype B consensus peptide, a HIV-1 subtype C consensus peptide, a HIV-1 subtype D consensus peptide, and any combination thereof.

### BRIEF DESCRIPTION OF THE DRAWINGS

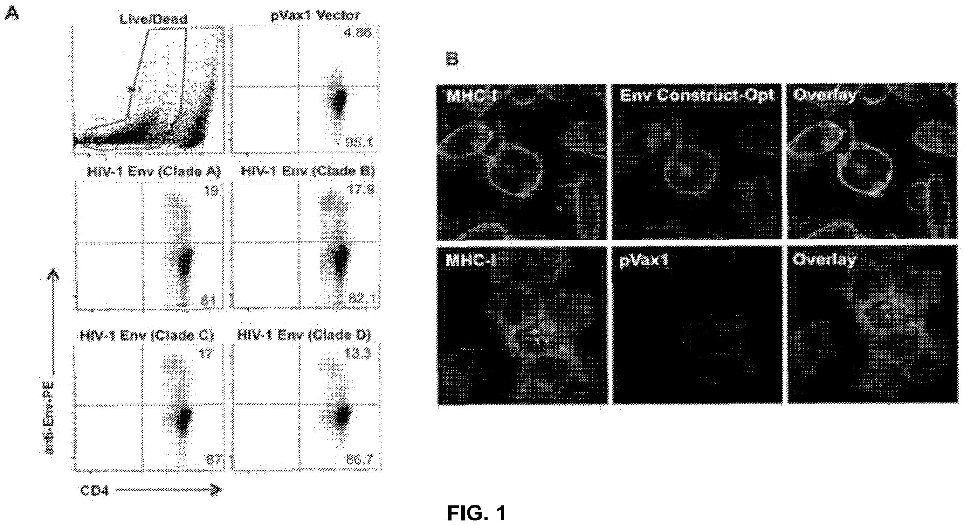

**Figure** 1. Optimized HIV-1 Env protein expression. (A) Jurkat T-cells were transfected with an HIV-1 Env-expressing plasmid and expression was determined by FACS. Cells were stained with anti-HIV core or control antibodies followed by PE-conjugated goat anti-mouse and human CD4-FITC treatment. (B) Immunofluorescent analysis of optimized envelope expression. Human RD cells were transfected with vaccine constructs and stained with monoclonal antibodies against MHC-I and HIV-1 Env prior to confocal analysis. MHC-I was used as a marker for cell surface expression as it is ubiquitously expressed on the surface of nucleated mammalian cells.

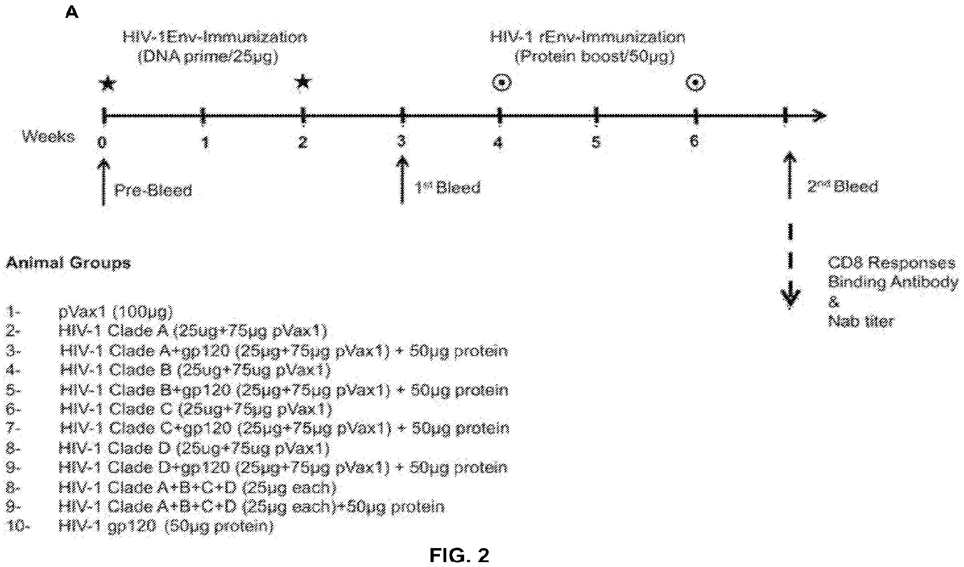

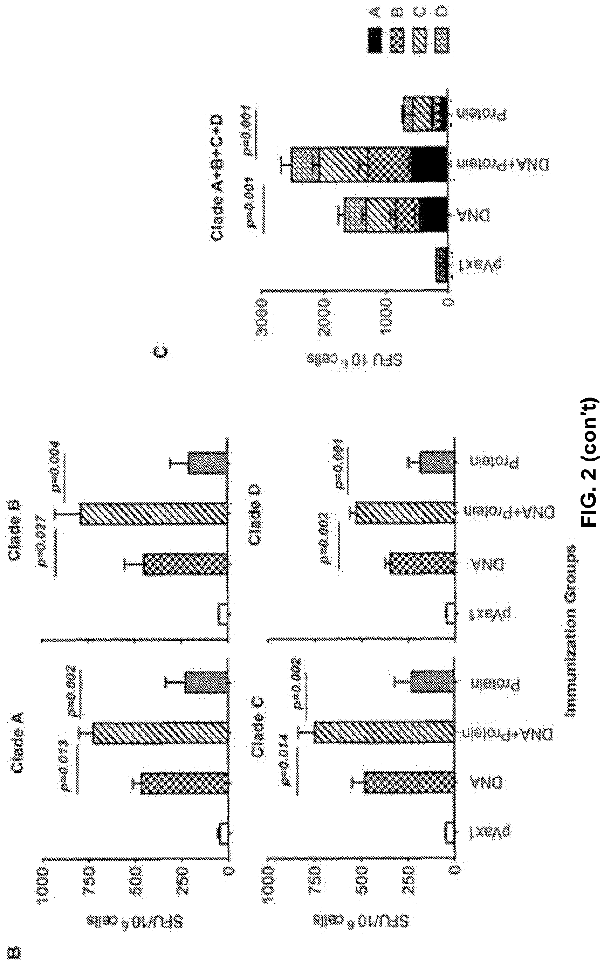

**Figure 2**. HIV-1 Env vaccines are potent inducers of cell-mediated immune response. (A) Design of animal groups for DNA prime-protein boost immunization study in BALB/c. The antigen-specific T-cell responses from a single plasmid (B) or combined plasmid (C) formulation were assessed by the IFN-γ ELIspot. Splenic T-cells were stimulated with BALB/c immunodominant Env peptide and IFN-γ spot forming cells were enumerated after overnight incubation. Results shown are the mean number of spot forming cells (SFC)± SD for four animals/group with control SFC counts with background peptide subtracted.

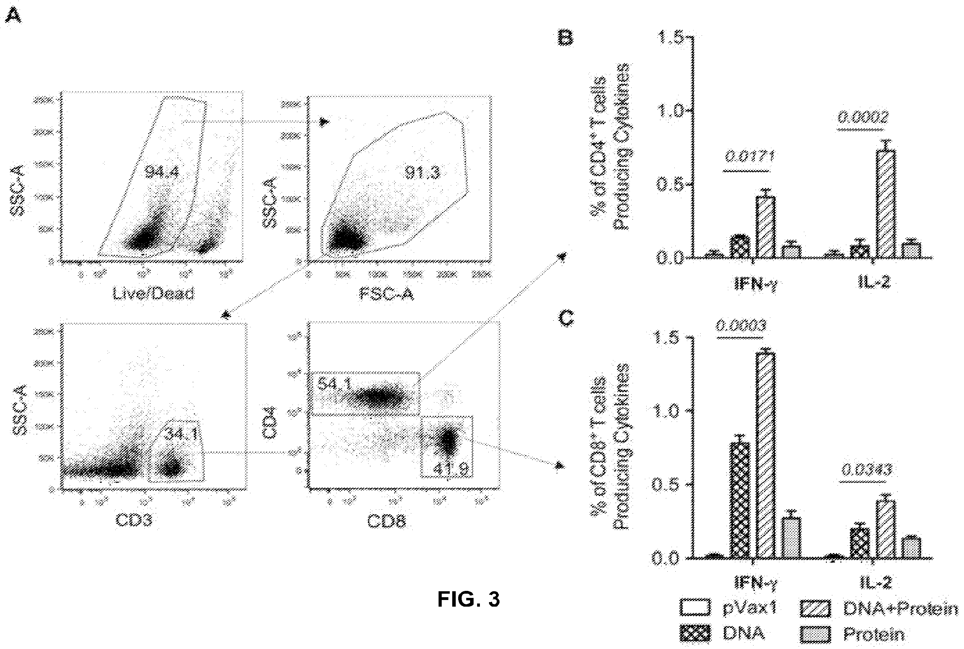

**Figure 3**. IFN-γ and IL-2 production in response to HIV-1 Env antigen. Intracellular cytokine staining with flow cytometry analysis of IFN-γ and IL-2 expressing, Env-specific CD8⁺/CD4⁺ splenic T cells stimulated with Env peptides. Mice were immunized with indicated Env constructs, and splenocytes were collected and cultured. (A) Representative flow cytometry data for splenocytes harvested from mice immunized with combined DNA and stimulated for five hours with the envelope peptide. (B&C) Bar graph showing the number of Env-specific IFN-γ and IL-2 expressing (B) CD4⁺ and (C) CD8⁺T-cell responses generated by in vitro stimulation as described in Materials & Methods. Results are the mean ± SD for 4 mice per group (n=4). Data presented in this figure are from one experiment representative of two performed.

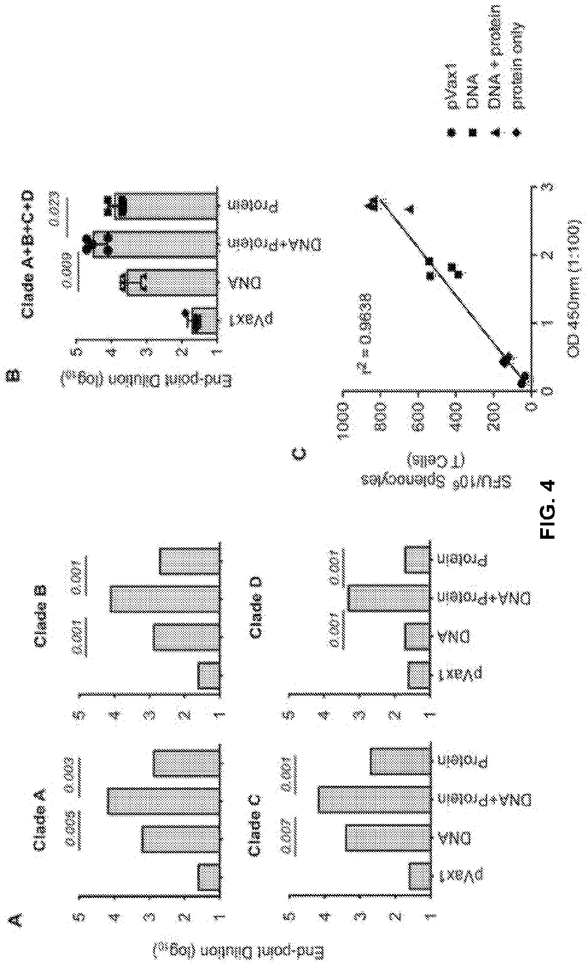

**Figure 4**. Characterization of antisera directed against HIV-1 Env. Binding of mouse antisera from DNA prime-protein boosts with subtypes A, B C and D envelope DNA and subtype B proteins. ELISA plates were coated with recombinant gp120 (subtype B) envelope glycoproteins. (A&B) End-point antigp120 IgG titers obtained from mice (n=4) immunized with different Env immunogens as indicated, data shown titers at day 35, one week after the second protein boost. C) Correlation between the binding antibody titers and SFU obtained by T-cell ELISpot assay.

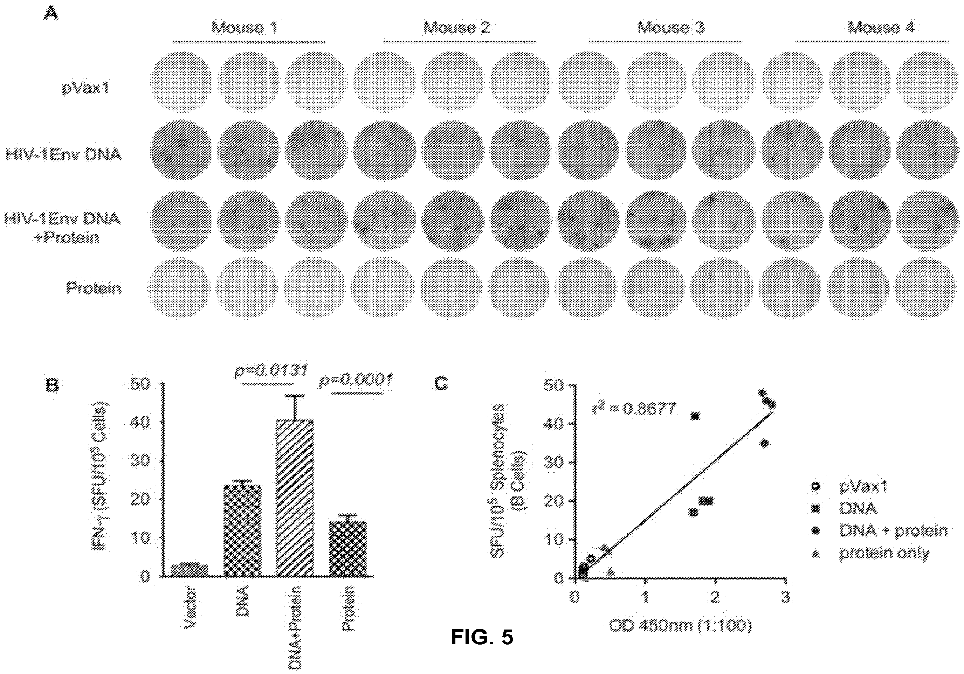

**Figure 5**. Detection of antibody secreting cells (ASCs). Groups of mice (n=4) were immunized with the indicated constructs. (A) 96-well plates were coated with goat anti-mouse IgG in PBS and blocked overnight at 4°C. Approximate number of IgG producing B-cells was determined by ELISpot assay. (B) Representative plots of two individual experiments are shown; error bars represent standard deviation of at least three replicate wells. (C) Correlation between the binding antibody titers and SFU obtained by B-cell ELISpot assay.

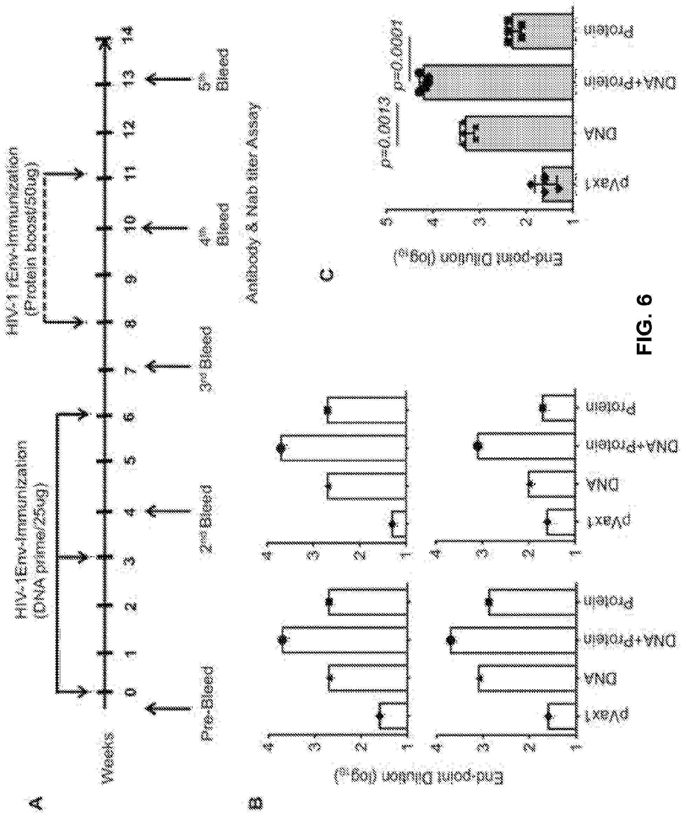

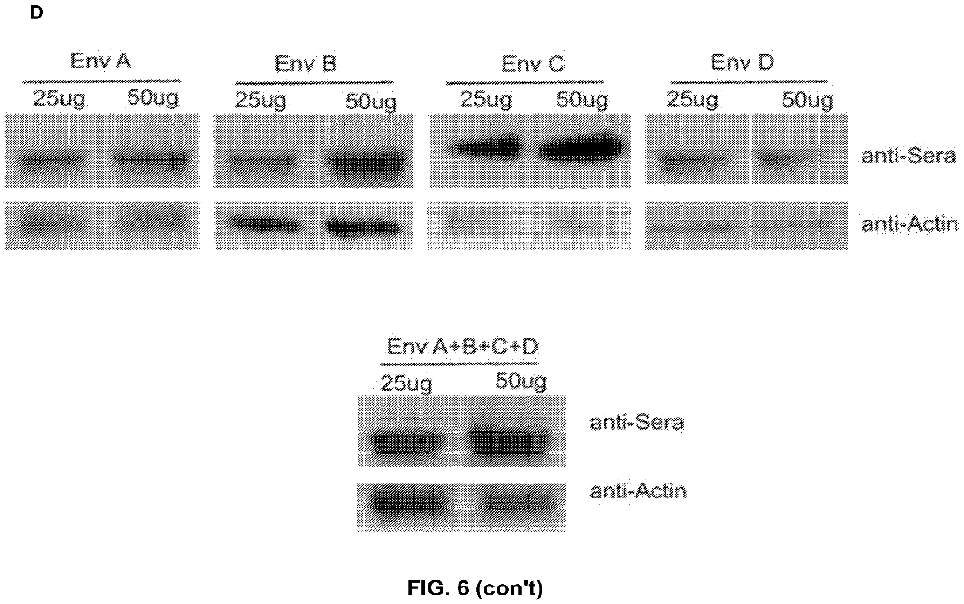

**Figure 6**. Guinea pig Immunization and antibody binding. (A) Timeline for the DNA prime-protein boost immunization study in guinea pigs. Serum samples from the immunized and control guinea pigs were obtained as indicated. (B&C) Anti-gp120 antibody-binding titers were determined by ELISA two weeks after the first protein boost (n=5). Data are presented as the mean endpoint titers ±SD. (D) Specificity of anti-gp120 IgG analyzed by Western blot analysis using sera from multi-clade prime and recombinant protein boost immunized guinea pigs. Cell lysates from 293T cells transiently transfected with HIV-1 Env plasmid (pHxB2) and were loaded onto 10% SDS-PAGE and were analyzed by Western blot using sera from HIV-1 Env as indicated immunized guinea pigs as the primary antibody at a dilution of 1:500. Sera were collected two weeks after the final protein immunization.

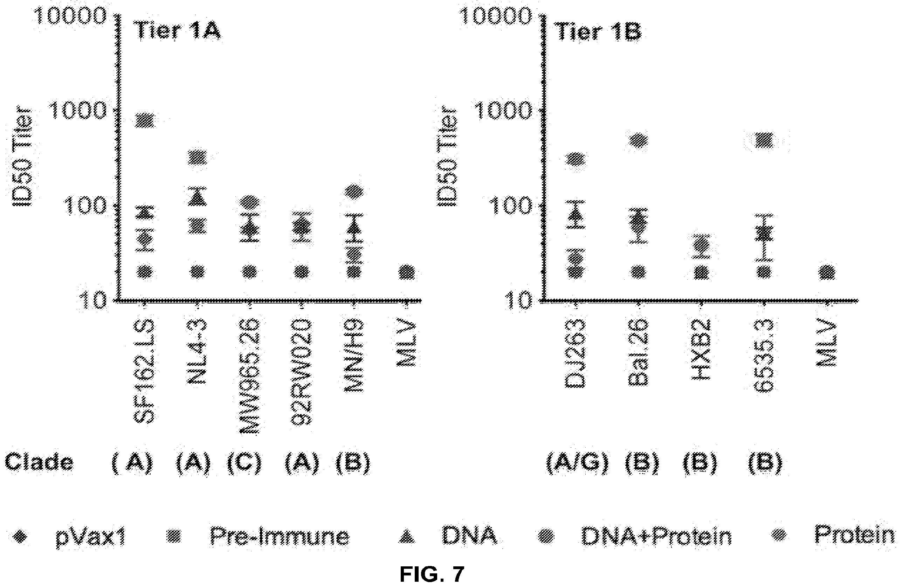

**Figure 7**. Neutralizing antibody titers against HIV-1 from immunized guinea pig sera. Guinea pig sera were collected two weeks after the last protein immunization for testing. The neutralization experiment was conducted in TZM-bl cells using a panel of Envelope tier 1 pseudo viruses as described in Materials and Methods. Neutralization titers were defined by the sera dilution that achieves 50% inhibition of viral isolates (ID50).

### DETAILED DESCRIPTION

The inventors have made the surprising discovery of a synthetic consensus DNA vaccine encoding HIV antigenic constructs derived from individual HIV-1 subtypes A, B, C and D in a DNA prime-protein boost regimen. These consensus DNA constructs can be optimized using the following plasmid-enhancement techniques: codon optimization, RNA optimization, leader sequence addition, plasmid production at high concentrations and the DNA was delivered by adaptive EP as previously described. The DNA prime can be followed by a protein boost with recombinant HIV gp120. Immune responses were measured by ELISA, B-cell ELISpot, T-cell ELISpot, and in a TZM-bl neutralization assay. The combination approach increased T cell and antibody functionality over these observed with either independent modality.

### 1. Definitions.

The terminology used herein is for the purpose of describing particular embodiments only and is not intended to be limiting. As used in the specification and the appended claims, the singular forms "a," "an" and "the" include plural referents unless the context clearly dictates otherwise.

For recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9, and 7.0 are explicitly contemplated.

"Consensus" or "Consensus Sequence" as used herein may mean a synthetic nucleic acid sequence, or corresponding polypeptide sequence, constructed based on analysis of an alignment of multiple subtypes of a particular antigen. The sequence may be used to induce broad immunity against multiple subtypes or sertypes of a particular antigen. Synthetic antigens, such as fusion proteins, may be manipulated to consensus sequences (or consensus antigens).

A "peptide" or "polypeptide" is a linked sequence of amino acids and can be natural, synthetic, or a modification or combination of natural and synthetic.

"Treatment" or "treating," when referring to protection of an animal from a disease, means preventing, suppressing, repressing, or completely eliminating the disease. Preventing the disease involves administering a composition of the present invention to an animal prior to onset of the disease. Suppressing the disease involves administering a composition of the present invention to an animal after induction of the disease but before its clinical appearance. Repressing the disease involves administering a composition of the present invention to an animal after clinical appearance of the disease.

"Substantially identical" can mean that a first and second amino acid sequence are at least 60%, 65%, 70%, 75%, 80%, 85%, 90%, 95%, 96%, 97%, 98%,or 99% over a region of 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 200, 300, 400, 500, 600, 700, 800, 900, 1000, 1100 amino acids.

A "variant" can mean means a peptide or polypeptide that differs in amino acid sequence by the insertion, deletion, or conservative substitution of amino acids, but retain at least one biological activity. Representative examples of "biological activity" include the ability to be bound by a specific antibody or to promote an immune response. Variant can also mean a protein with an amino acid sequence that is substantially identical to a referenced protein with an amino acid sequence that retains at least one biological activity. A conservative substitution of an amino acid, i.e., replacing an amino acid with a different amino acid of similar properties (e.g., hydrophilicity, degree and distribution of charged regions) is recognized in the art as typically involving a minor change. These minor changes can be identified, in part, by considering the hydropathic index of amino acids. See Kyte et al., J. Mol. Biol. 157:105-132 (1982). The hydropathic index of an amino acid is based on a consideration of its hydrophobicity and charge. It is known in the art that amino acids of similar hydropathic indexes can be substituted and still retain protein function. In one aspect, amino acids having hydropathic indexes of ±2 are substituted. The hydrophilicity of amino acids can also be used to reveal substitutions that would result in proteins retaining biological function. A consideration of the hydrophilicity of amino acids in the context of a peptide permits calculation of the greatest local average hydrophilicity of that peptide, a useful measure that has been reported to correlate well with antigenicity and immunogenicity, as discussed in U.S. Patent No. 4,554,101, which is fully incorporated herein by reference. Substitution of amino acids having similar hydrophilicity values can result in peptides retaining biological activity, for example immunogenicity, as is understood in the art. Substitutions can be performed with amino acids having hydrophilicity values within ±2 of each other. Both the hyrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties.

### 2. Composition for Priming and Boosting HIV Immunological Response

Provided herein is a composition comprising a first vaccine and a second vaccine for priming and boosting an immune response to HIV. The first vaccine is comprised of at least 1, at least 2, at least 3, at least 4, at least 5, at least 6, at least 7, at least 8, at least 9, or at least 10 nucleic acids comprising an antigen. The second vaccine comprises at least 1, at least 2, at least 3, at least 4, at least 5, at least 6, at least 7, at least 8, at least 9, or at least 10 antigenic peptides. The first vaccine is administered day 1 of the vaccination regimen. The first vaccine may be given in multiple doses. The first vaccine can be administered a second time within 12 hours, 24 hours, 36 hours, or 48 hours of the first administration of the first vaccine. The first vaccine may be a priming vaccination.

The second vaccine can be administered to boost the first vaccine. The second vaccine maybe administered a first time 48 hours, 60 hours, 72 hours, 84 hours, 90 hours, 1.5 weeks, 2 weeks, 2.5 weeks, 3.0 weeks, 3.5 weeks, 4.0 weeks, 4.5 weeks, 5.0 weeks, 5.5 weeks, 6.0 weeks, 6.5 weeks, 7.0 weeks, 7.5 weeks, 8.0 weeks, 8.5 weeks, 9.0 weeks, 9.5 weeks, 10.0 weeks, 10.5 weeks, 11.0 weeks, 11.5 weeks, 12.0 weeks, 12.5 weeks, 13.0 weeks, 13.5 weeks, 14.0 weeks, 14.5 weeks or 15.0 weeks after the administration of the first vaccine. The second vaccine may be in administered in multiple doses. The second vaccine may be administered a second time after 60 hours, 72 hours, 84 hours, 90 hours, 1.5 weeks, 2 weeks, 2.5 weeks, 3.0 weeks, 3.5 weeks, 4.0 weeks, 4.5 weeks, 5.0 weeks, 5.5 weeks, 6.0 weeks, 6.5 weeks, 7.0 weeks, 7.5 weeks, 8.0 weeks, 8.5 weeks, 9.0 weeks, 9.5 weeks, 10.0 weeks, 10.5 weeks, 11.0 weeks, 11.5 weeks, 12.0 weeks, 12.5 weeks, 13.0 weeks, 13.5 weeks, 14.0 weeks, 14.5 weeks or 15.0 weeks, or 15.5 weeks after the first administration of the second vaccine.

The vaccine can induce antigen-specific T cells that inhibit antigen-specific T cell function. The combination of the first vaccine comprising a DNA encoding the antigen and a second vaccine comprising antigen for boosting the immune response to the first vaccine induces an immunological response efficiently against specific antigens far better than either a vaccine comprising an antigen or its corresponding DNA alone. The vaccine can further enhance MHC Class II presentation and expression for iTreg cell induction.

### a. Antigen

The composition may comprise an antigen. The antigen is encoded by a nucleic acid sequence. The nucleic acid sequence may be DNA or RNA. The nucleic acid may encode an antigen or a variant thereof. The antigen can be the same antigen or a different antigen between the first and the second vaccine. The antigen can be an antigen isolated from human immunodeficiency virus (HIV). The HIV antigens can include modified consensus sequences for immunogens. Genetic modifications including codon optimization, RNA optimization, and the addition of a high efficient immunoglobin leader sequence to increase the immunogenicity of constructs can be included in the modified consensus sequences. The novel immunogens can be designed to elicit stronger and broader cellular immune responses than a corresponding codon optimized immunogens.

The antigen of the first vaccine may be the same antigen across different subtypes of HIV. The first vaccine may comprise 1 or more, 2 or more, 3 or more, 4 or more, or 5 or more DNA sequences encoding a particular protein sequence isolated from HIV subtypes A, B, C, D, or other HIV subtypes, or a combination or variant thereof. The antigen of the first vaccine may be the same antigen across different subtypes of HIV. The first vaccine may comprise 1 or more, 2 or more, 3 or more, 4 or more, or 5 or more consensus DNA sequences encoding a particular protein sequence isolated from HIV subtypes A, B, C, D, or other HIV subtypes, or a combination or variant thereof. The first vaccine may comprise a DNA sequence encoding a particular protein sequence isolated from HIV subtype A, a second DNA sequence encoding a particular protein sequence isolated from HIV subtype B, a third DNA sequence encoding a particular protein sequence isolated from HIV subtype C, a fourth DNA sequence encoding a particular protein sequence isolated from HIV subtype D, or a combination thereof. The first vaccine may comprise a consensus DNA sequence encoding a particular protein sequence isolated from HIV subtype A, a second consensus DNA sequence encoding a particular protein sequence isolated from HIV subtype B, a third consensus DNA sequence encoding a particular protein sequence isolated from HIV subtype C, a fourth consensus DNA sequence encoding a particular protein sequence isolated from HIV subtype D, or a combination thereof. The first vaccine may comprise a consensus DNA sequence or variant thereof encoding a particular HIV subtype A protein sequence or variant thereof, a second consensus DNA sequence or variant thereof encoding a particular HIV subtype B protein sequence or variant thereof, a third consensus DNA sequence or variant thereof encoding a particular HIV subtype C protein sequence or variant thereof, a fourth consensus DNA sequence or variant thereof encoding a particular HIV subtype D protein sequence or variant thereof.

The second vaccine may comprise 1 or more, 2 or more, 3 or more, 4 or more, 5 or more antigenic peptide sequences isolated from HIV subtypes A, B, C, D, or other HIV subtypes, or a combination or variant thereof. The second vaccine may comprise 1 or more, 2 or more, 3 or more, 4 or more, 5 or more consensus antigenic peptide sequences isolated from HIV subtypes A, B, C, D, or other HIV subtypes, or a combination or variant thereof. The second vaccine may comprise a antigenic peptide that is the same or different from the DNA encoded peptides of the first vaccine. The second vaccine may comprise a particular protein sequence isolated from HIV subtype A, subtype B, subtype C, subtype D or other HIV subtypes. The second vaccine may comprise a particular consensus protein sequence isolated from HIV subtype A, subtype B, subtype C, subtype D or other HIV subtypes.

In some embodiments, the HIV antigen can be a subtype A consensus envelope DNA sequence construct, an IgE leader sequence linked to a consensus sequence for Subtype A envelope protein, or a subtype A consensus Envelope protein sequence.

In other embodiments, the HIV antigen can be a subtype B consensus envelope DNA sequence construct, an IgE leader sequence linked to a consensus sequence for Subtype B envelope protein, or an subtype B consensus Envelope protein sequence.

In still other embodiments, the HIV antigen can be a subtype C consensus envelope DNA sequence construct, an IgE leader sequence linked to a consensus sequence for subtype C envelope protein, or a subtype C consensus envelope protein sequence.

In further embodiments, the HIV antigen can be a subtype D consensus envelope DNA sequence construct, an IgE leader sequence linked to a consensus sequence for Subtype D envelope protein, or a subtype D consensus envelope protein sequence.

In some embodiments, the HIV antigen can be a subtype A Nef-Rev consensus envelope DNA sequence construct, an IgE leader sequence linked to a consensus sequence for Subtype A Nef-Rev protein, or a Subtype A Nef-Rev consensus protein sequence.

In some embodiments, the HIV antigen can be a subtype B Nef-Rev consensus envelope DNA sequence construct, an IgE leader sequence linked to a consensus sequence for Subtype B Nef-Rev protein, or a Subtype B Nef-Rev consensus protein sequence.

In some embodiments, the HIV antigen can be a subtype C Nef-Rev consensus envelope DNA sequence construct, an IgE leader sequence linked to a consensus sequence for Subtype C Nef-Rev protein, or a Subtype C Nef-Rev consensus protein sequence.

In some embodiments, the HIV antigen can be a subtype D Nef-Rev consensus envelope DNA sequence construct, an IgE leader sequence linked to a consensus sequence for Subtype D Nef-Rev protein, or a Subtype D Nef-Rev consensus protein sequence.

In other embodiments, the HIV antigen can be a Gag consensus DNA sequence of subtype A, B, C and D DNA sequence construct, an IgE leader sequence linked to a consensus sequence for Gag consensus subtype A, B, C and D protein, or a consensus Gag subtype A, B, C and D protein sequence.

In still other embodiments, the HIV antigen can be a MPol DNA sequence or a MPol protein sequence. The HIV antigen can be nucleic acid or amino acid sequences of Env A, Env B, Env C, Env D, B Nef-Rev , Gag, or any combination thereof.

In other embodiments, the HIV antigen may be a DNA sequence or consensus sequence of subtype A, B, C, or Dencoding gp140 or consensus gp140 protein. In other embodiments, the HIV antigen may be a DNA sequence or consensus sequence of subtype A, B, C, or D encoding gp140 or consensus gp120 protein. In other embodiments, the HIV antigen gp140 peptide sequence or gp140 consensus peptide sequence of subtype A, B, C, or D. In other embodiments, the HIV antigen gp120 peptide sequence or gp140 consensus peptide sequence of subtype A, B, C, or D.

The antigen can affect a mammal, which can be a human, chimpanzee, dog, cat, horse, cow, mouse, or rat. The antigen can be contained in a protein from a mammal, which can be a human, chimpanzee, dog, cat, horse, cow, pig, sheep, mouse, or rat.

### b. DNA

The composition may comprise the DNA. Also provided herein is a DNA that encodes the antigen as described above. The DNA can include an encoding sequence that encodes the antigen. The DNA can also include additional sequences that encode linker or tag sequences that are linked to the antigen by a peptide bond.

### c. Vector

The composition may comprise a vector that includes the DNA encoding the antigen. The vector can be capable of expressing the antigen. The vector may be an expression construct, which is generally a plasmid that is used to introduce a specific gene into a target cell. Once the expression vector is inside the cell, the protein that is encoded by the gene is produced by the cellular-transcription and translation machinery ribosomal complexes. The plasmid is frequently engineered to contain regulatory sequences that act as enhancer and promoter regions and lead to efficient transcription of the gene carried on the expression vector. The vectors of the present invention express large amounts of stable messenger RNA, and therefore proteins.

The vectors may have expression signals such as a strong promoter, a strong termination codon, adjustment of the distance between the promoter and the cloned gene, and the insertion of a transcription termination sequence and a PTIS (portable translation initiation sequence).

### i. Expression vectors

The vector may be circular plasmid or a linear nucleic acid vaccine. The circular plasmid and linear nucleic acid are capable of directing expression of a particular nucleotide sequence in an appropriate subject cell. The vector may have a promoter operably linked to the antigen-encoding nucleotide sequence, which may be operably linked to termination signals. The vector may also contain sequences required for proper translation of the nucleotide sequence. The vector comprising the nucleotide sequence of interest may be chimeric, meaning that at least one of its components is heterologous with respect to at least one of its other components. The expression of the nucleotide sequence in the expression cassette may be under the control of a constitutive promoter or of an inducible promoter which initiates transcription only when the host cell is exposed to some particular external stimulus. In the case of a multicellular organism, the promoter can also be specific to a particular tissue or organ or stage of development.

### ii. Circular and Linear Vectors

The vector may be circular plasmid, which may transform a target cell by integration into the cellular genome or exist extrachromosomally (e.g. autonomous replicating plasmid with an origin of replication).

The vector can be pVAX, pcDNA3.0, or provax, or any other expression vector capable of expressing the DNA and enabling a cell to translate the sequence to a antigen that is recognized by the immune system. The vector can be combined with antigen at a mass ratio of between 5:1 and 1:5, or of between 1:1 and 2:1.

Also provided herein is a linear nucleic acid vaccine, or linear expression cassette ("LEC"), that is capable of being efficiently delivered to a subject via electroporation and expressing one or more desired antigens. The LEC may be any linear DNA devoid of any phosphate backbone. The DNA may encode one or more antigens. The LEC may contain a promoter, an intron, a stop codon, a polyadenylation signal. The expression of the antigen may be controlled by the promoter. The LEC may not contain any antibiotic resistance genes and/or a phosphate backbone. The LEC may not contain other nucleic acid sequences unrelated to the desired antigen gene expression.

The LEC may be derived from any plasmid capable of being linearized. The plasmid may be capable of expressing the antigen. The plasmid may be pNP (Puerto Rico/34) or pM2 (New Caledonia/99). See Figure 1. The plasmid may be pVAX, pcDNA3.0, or provax, or any other expression vector capable of expressing the DNA and enabling a cell to translate the sequence to a antigen that is recognized by the immune system.

The LEC may be pcrM2. The LEC may be pcrNP. pcrNP and pcrMR may be derived from pNP (Puerto Rico/34) and pM2 (New Caledonia/99), respectively. See Figure 34. The LEC may be combined with antigen at a mass ratio of between 5:1 and 1:5, or of between 1:1 to 2:1.

### iii. Promoter, Intron, Stop codon, and Polyadenylation signal

The vector may have a promoter. A promoter may be any promoter that is capable of driving gene expression and regulating expression of the isolated nucleic acid. Such a promoter is a cis-acting sequence element required for transcription via a DNA dependent RNA polymerase, which transcribes the antigen sequence described herein. Selection of the promoter used to direct expression of a heterologous nucleic acid depends on the particular application. The promoter may be positioned about the same distance from the transcription start in the vector as it is from the transcription start site in its natural setting. However, variation in this distance may be accommodated without loss of promoter function.

The promoter may be operably linked to the nucleic acid sequence encoding the antigen and signals required for efficient polyadenylation of the transcript, ribosome binding sites, and translation termination. The promoter may be a CMV promoter, SV40 early promoter, SV40 later promoter, metallothionein promoter, murine mammary tumor virus promoter, Rous sarcoma virus promoter, polyhedrin promoter, or another promoter shown effective for expression in eukaryotic cells.

The vector may include an enhancer and an intron with functional splice donor and acceptor sites. The vector may contain a transcription termination region downstream of the structural gene to provide for efficient termination. The termination region may be obtained from the same gene as the promoter sequence or may be obtained from different genes.

### d. Other Components of Vaccine-Adjuvants, Excipients

The composition may further comprise a pharmaceutically acceptable excipient. The pharmaceutically acceptable excipient can be functional molecules as vehicles, adjuvants, carriers, or diluents. The pharmaceutically acceptable excipient can be a transfection facilitating agent, which can include surface active agents, such as immune-stimulating complexes (ISCOMS), Freunds incomplete adjuvant, LPS analog including monophosphoryl lipid A, muramyl peptides, quinone analogs, vesicles such as squalene and squalene, hyaluronic acid, lipids, liposomes, calcium ions, viral proteins, polyanions, polycations, or nanoparticles, or other known transfection facilitating agents.

The transfection facilitating agent is a polyanion, polycation, including poly-L-glutamate (LGS), or lipid. The transfection facilitating agent is poly-L-glutamate, and the poly-L-glutamate is may be present in the vaccine at a concentration less than 6 mg/ml. The transfection facilitating agent may also include surface active agents such as immune-stimulating complexes (ISCOMS), Freunds incomplete adjuvant, LPS analog including monophosphoryl lipid A, muramyl peptides, quinone analogs and vesicles such as squalene and squalene, and hyaluronic acid may also be used administered in conjunction with the genetic construct. The DNA plasmid vaccines may also include a transfection facilitating agent such as lipids, liposomes, including lecithin liposomes or other liposomes known in the art, as a DNA-liposome mixture (see for example WO9324640), calcium ions, viral proteins, polyanions, polycations, or nanoparticles, or other known transfection facilitating agents. The transfection facilitating agent is a polyanion, polycation, including poly-L-glutamate (LGS), or lipid. Concentration of the transfection agent in the vaccine is less than 4 mg/ml, less than 2 mg/ml, less than 1 mg/ml, less than 0.750 mg/ml, less than 0.500 mg/ml, less than 0.250 mg/ml, less than 0.100 mg/ml, less than 0.050 mg/ml, or less than 0.010 mg/ml.

The pharmaceutically acceptable excipient can be an adjuvant. The adjuvant can be other genes that are expressed in alternative plasmid or are delivered as proteins in combination with the plasmid above in the vaccine. The adjuvant may be selected from the group consisting of: α-interferon(IFN- α), β-interferon (IFN-β), γ-interferon, platelet derived growth factor (PDGF), TNFα, TNPβ, GM-CSF, epidermal growth factor (EGF), cutaneous T cell-attracting chemokine (CTACK), epithelial thymus-expressed chemokine (TECK), mucosae-associated epithelial chemokine (MEC), IL-12, IL-15, MHC, CD80,CD86 including IL-15 having the signal sequence deleted and optionally including the signal peptide from IgE. The adjuvant can be IL-12, IL-15, IL-28, CTACK, TECK, platelet derived growth factor (PDGF), TNFα, TNFβ, GM-CSF, epidermal growth factor (EGF), IL-1, IL-2, IL-4, IL-5, IL-6, IL-10, IL-12, IL-18, or a combination thereof.

Other genes that can be useful adjuvants include those encoding: MCP-1, MIP-la, MIP-1p, IL-8, RANTES, L-selectin, P-selectin, E-selectin, CD34, GlyCAM-1, MadCAM-1, LFA-1, VLA-1, Mac-1, pl50.95, PECAM, ICAM-1, ICAM-2, ICAM-3, CD2, LFA-3, M-CSF, G-CSF, IL-4, mutant forms of IL-18, CD40, CD40L, vascular growth factor, fibroblast growth factor, IL-7, nerve growth factor, vascular endothelial growth factor, Fas, TNF receptor, Flt, Apo-1, p55, WSL-1, DR3, TRAMP, Apo-3, AIR, LARD, NGRF, DR4, DR5, KILLER, TRAIL-R2, TRICK2, DR6, Caspase ICE, Fos, c-jun, Sp-1, Ap-1, Ap-2, p38, p65Rel, MyD88, IRAK, TRAF6, IkB, Inactive NIK, SAP K, SAP-1, JNK, interferon response genes, NFkB, Bax, TRAIL, TRAILrec, TRAILrecDRC5, TRAIL-R3, TRAIL-R4, RANK, RANK LIGAND, Ox40, Ox40 LIGAND, NKG2D, MICA, MICB, NKG2A, NKG2B, NKG2C, NKG2E, NKG2F, TAP1, TAP2 and functional fragments thereof.

The vaccine may further comprise a genetic vaccine facilitator agent as described in U.S. Serial No. 021,579 filed April 1, 1994, which is fully incorporated by reference.

The vaccine can be formulated according to the mode of administration to be used. An injectable vaccine pharmaceutical composition can be sterile, pyrogen free and particulate free. An isotonic formulation or solution can be used. Additives for isotonicity can include sodium chloride, dextrose, mannitol, sorbitol, and lactose. The vaccine can comprise a vasoconstriction agent. The isotonic solutions can include phosphate buffered saline. Vaccine can further comprise stabilizers including gelatin and albumin. The stabilizers can allow the formulation to be stable at room or ambient temperature for extended periods of time, including LGS or polycations or polyanions.

### 3. Method of vaccination

Provided herein is a method of vaccinating a patient to treat or prevent HIV infection using the composition. The method of vaccinating a patient comprises administering the composition to a subject in need thereof. The first vaccine can efficiently deliver antigen to a subject in need thereof for immune stimulation via a priming vaccination. A subject's immune system may be efficiently induced against specific antigens by administering a priming DNA that encodes the antigen followed by a boost antigenic peptide. The DNA vaccine, which may contain an antigen-encoding circular plasmid or a linear nucleic acid, can elicit antigen-specific antibody responses, which are sustainable for longer periods of time as compared to plasmid-based vaccines, when efficiently delivered to a subject. The second vaccine may be a boosting vaccine to the first vaccine and comprise the same or different antigenic peptide as the first vaccine.

The herein described composition may be administered to a subject so as to provide a longer lasting antigen-specific immune response that is well tolerated by the subject population. The present invention is also directed to a number of antigens that can be expressed from the DNA.

The dose of the first and second vaccine can be between 1 µg to 10 mg active component/kg body weight/time, and can be 20 µg to 10 mg component/kg body weight/time. The first vaccine is administered day 1 of the vaccination regimen. The first vaccine may be given in multiple doses. The first vaccine can be administered a second time within 12 hours, 24 hours, 36 hours, or 48 hours of the first administration of the first vaccine. The first vaccine may be a priming vaccination.

The second vaccine can be administered to boost the first vaccine. The second vaccine maybe administered a first time 48 hours, 60 hours, 72 hours, 84 hours, 90 hours, 1.5 weeks, 2 weeks, 2.5 weeks, 3.0 weeks, 3.5 weeks, 4.0 weeks, 4.5 weeks, 5.0 weeks, 5.5 weeks, 6.0 weeks, 6.5 weeks, 7.0 weeks, 7.5 weeks, 8.0 weeks, 8.5 weeks, 9.0 weeks, 9.5 weeks, 10.0 weeks, 10.5 weeks, 11.0 weeks, 11.5 weeks, 12.0 weeks, 12.5 weeks, 13.0 weeks, 13.5 weeks, 14.0 weeks, 14.5 weeks or 15.0 weeks after the administration of the first vaccine. The second vaccine may be in administered in multiple doses. The second vaccine may be administered a second time after 60 hours, 72 hours, 84 hours, 90 hours, 1.5 weeks, 2 weeks, 2.5 weeks, 3.0 weeks, 3.5 weeks, 4.0 weeks, 4.5 weeks, 5.0 weeks, 5.5 weeks, 6.0 weeks, 6.5 weeks, 7.0 weeks, 7.5 weeks, 8.0 weeks, 8.5 weeks, 9.0 weeks, 9.5 weeks, 10.0 weeks, 10.5 weeks, 11.0 weeks, 11.5 weeks, 12.0 weeks, 12.5 weeks, 13.0 weeks, 13.5 weeks, 14.0 weeks, 14.5 weeks or 15.0 weeks, or 15.5 weeks after the first administration of the second vaccine.

The number of vaccine doses for effective treatment can be 1, 2, 3, 4, 5, 6, 7, 8, 9, or 10.

### a. Administration

The composition can be formulated in accordance with standard techniques well known to those skilled in the pharmaceutical art. Such compositions can be administered in dosages and by techniques well known to those skilled in the medical arts taking into consideration such factors as the age, sex, weight, and condition of the particular subject, and the route of administration. The subject can be a mammal, such as a human, a horse, a cow, a pig, a sheep, a cat, a dog, a rat, or a mouse.

The composition can be administered prophylactically or therapeutically. In prophylactic administration, the vaccines can be administered in an amount sufficient to induce iTreg responses. In therapeutic applications, the vaccines are administered to a subject in need thereof in an amount sufficient to elicit a therapeutic effect. An amount adequate to accomplish this is defined as "therapeutically effective dose." Amounts effective for this use will depend on, e.g., the particular composition of the vaccine regimen administered, the manner of administration, the stage and severity of the disease, the general state of health of the patient, and the judgment of the prescribing physician.

The composition can be administered by methods well known in the art as described in Donnelly et al. (Ann. Rev. Immunol. 15:617-648 (1997)); Felgner et al. (U.S. Pat. No. 5,580,859, issued Dec. 3, 1996); Felgner (U.S. Pat. No. 5,703,055, issued Dec. 30, 1997); and Carson et al. (U.S. Pat. No. 5,679,647, issued Oct. 21, 1997), the contents of all of which are incorporated herein by reference in their entirety. The DNA of the vaccine can be complexed to particles or beads that can be administered to an individual, for example, using a vaccine gun. One skilled in the art would know that the choice of a pharmaceutically acceptable carrier, including a physiologically acceptable compound, depends, for example, on the route of administration of the expression vector.

The composition can be delivered via a variety of routes. Typical delivery routes include parenteral administration, e.g., intradermal, intramuscular or subcutaneous delivery. Other routes include oral administration, intranasal, and intravaginal routes. For the DNA of the vaccine in particular, the vaccine can be delivered to the interstitial spaces of tissues of an individual (Felgner et al., U.S. Pat. Nos. 5,580,859 and 5,703,055, the contents of all of which are incorporated herein by reference in their entirety). The vaccine can also be administered to muscle, or can be administered via intradermal or subcutaneous injections, or transdermally, such as by iontophoresis. Epidermal administration of the vaccine can also be employed. Epidermal administration can involve mechanically or chemically irritating the outermost layer of epidermis to stimulate an immune response to the irritant (Carson et al., U.S. Pat. No. 5,679,647, the contents of which are incorporated herein by reference in its entirety).

The composition can also be formulated for administration via the nasal passages. Formulations suitable for nasal administration, wherein the carrier is a solid, can include a coarse powder having a particle size, for example, in the range of about 10 to about 500 microns which is administered in the manner in which snuff is taken, i.e., by rapid inhalation through the nasal passage from a container of the powder held close up to the nose. The formulation can be a nasal spray, nasal drops, or by aerosol administration by nebulizer. The formulation can include aqueous or oily solutions of the vaccine.

The composition can be a liquid preparation such as a suspension, syrup or elixir. The vaccine can also be a preparation for parenteral, subcutaneous, intradermal, intramuscular or intravenous administration (e.g., injectable administration), such as a sterile suspension or emulsion.

The composition can be incorporated into liposomes, microspheres or other polymer matrices (Felgner et al., U.S. Pat. No. 5,703,055; Gregoriadis, Liposome Technology, Vols. Ito III (2nd ed. 1993), the contents of which are incorporated herein by reference in their entirety). Liposomes can consist of phospholipids or other lipids, and can be nontoxic, physiologically acceptable and metabolizable carriers that are relatively simple to make and administer.

The composition can be administered via electroporation, such as by a method described in U.S. Patent No. 7,664,545, the contents of which are incorporated herein by reference. The electroporation can be by a method and/or apparatus described in U.S. Patent Nos. 6,302,874; 5,676,646; 6,241,701; 6,233,482; 6,216,034; 6,208,893; 6,192,270; 6,181,964; 6,150,148; 6,120,493; 6,096,020; 6,068,650; and 5,702,359, the contents of which are incorporated herein by reference in their entirety. The electroporation may be carried out via a minimally invasive device.

The minimally invasive electroporation device ("MID") may be an apparatus for injecting the vaccine described above and associated fluid into body tissue. The device may comprise a hollow needle, DNA cassette, and fluid delivery means, wherein the device is adapted to actuate the fluid delivery means in use so as to concurrently (for example, automatically) inject DNA into body tissue during insertion of the needle into the said body tissue. This has the advantage that the ability to inject the DNA and associated fluid gradually while the needle is being inserted leads to a more even distribution of the fluid through the body tissue. The pain experienced during injection may be reduced due to the distribution of the DNA being injected over a larger area.

The MID may inject the composition into tissue without the use of a needle. The MID may inject the vaccine as a small stream or jet with such force that the vaccine pierces the surface of the tissue and enters the underlying tissue and/or muscle. The force behind the small stream or jet may be provided by expansion of a compressed gas, such as carbon dioxide through a micro-orifice within a fraction of a second. Examples of minimally invasive electroporation devices, and methods of using them, are described in published U.S. Patent Application No. 20080234655; U.S. Patent No. 6,520,950; U.S. Patent No. 7,171,264; U.S. Patent No. 6,208,893; U.S. Patent NO. 6,009,347; U.S. Patent No. 6,120,493; U.S. Patent No. 7,245,963; U.S. Patent No. 7,328,064; and U.S. Patent No. 6,763,264, the contents of each of which are herein incorporated by reference.

The MID may comprise an injector that creates a high-speed jet of liquid that painlessly pierces the tissue. Such needle-free injectors are commercially available. Examples of needle-free injectors that can be utilized herein include those described in U.S. Patent Nos. 3,805,783; 4,447,223; 5,505,697; and 4,342,310, the contents of each of which are herein incorporated by reference.

A desired composition in a form suitable for direct or indirect electrotransport may be introduced (e.g., injected) using a needle-free injector into the tissue to be treated, usually by contacting the tissue surface with the injector so as to actuate delivery of a jet of the agent, with sufficient force to cause penetration of the vaccine into the tissue. For example, if the tissue to be treated is mucosa, skin or muscle, the agent is projected towards the mucosal or skin surface with sufficient force to cause the agent to penetrate through the stratum corneum and into dermal layers, or into underlying tissue and muscle, respectively.

Needle-free injectors are well suited to deliver vaccines to all types of tissues, particularly to skin and mucosa. In some embodiments, a needle-free injector may be used to propel a liquid that contains the vaccine to the surface and into the subject's skin or mucosa. Representative examples of the various types of tissues that can be treated using the invention methods include pancreas, larynx, nasopharynx, hypopharynx, oropharynx, lip, throat, lung, heart, kidney, muscle, breast, colon, prostate, thymus, testis, skin, mucosal tissue, ovary, blood vessels, or any combination thereof.

The MID may have needle electrodes that electroporate the tissue. By pulsing between multiple pairs of electrodes in a multiple electrode array, for example set up in rectangular or square patterns, provides improved results over that of pulsing between a pair of electrodes. Disclosed, for example, in U.S. Patent No. 5,702,359 entitled "Needle Electrodes for Mediated Delivery of Drugs and Genes" is an array of needles wherein a plurality of pairs of needles may be pulsed during the therapeutic treatment. In that application, which is incorporated herein by reference as though fully set forth, needles were disposed in a circular array, but have connectors and switching apparatus enabling a pulsing between opposing pairs of needle electrodes. A pair of needle electrodes for delivering recombinant expression vectors to cells may be used. Such a device and system is described in U.S. Patent No. 6,763,264, the contents of which are herein incorporated by reference. Alternatively, a single needle device may be used that allows injection of the DNA and electroporation with a single needle resembling a normal injection needle and applies pulses of lower voltage than those delivered by presently used devices, thus reducing the electrical sensation experienced by the patient.

The MID may comprise one or more electrode arrays. The arrays may comprise two or more needles of the same diameter or different diameters. The needles may be evenly or unevenly spaced apart. The needles may be between 0.005 inches and 0.03 inches, between 0.01 inches and 0.025 inches; or between 0.015 inches and 0.020 inches. The needle may be 0.0175 inches in diameter. The needles may be 0.5 mm, 1.0 mm, 1.5 mm, 2.0 mm, 2.5 mm, 3.0 mm, 3.5 mm, 4.0 mm, or more spaced apart.

The MID may consist of a pulse generator and a two or more-needle vaccine injectors that deliver the vaccine and electroporation pulses in a single step. The pulse generator may allow for flexible programming of pulse and injection parameters via a flash card operated personal computer, as well as comprehensive recording and storage of electroporation and patient data. The pulse generator may deliver a variety of volt pulses during short periods of time. For example, the pulse generator may deliver three 15 volt pulses of 100 ms in duration. An example of such a MID is the Elgen 1000 system by Inovio Biomedical Corporation, which is described in U.S. Patent No. 7,328,064, the contents of which are herein incorporated by reference.

The MID may be a CELLECTRA (Inovio Pharmaceuticals, Blue Bell PA) device and system, which is a modular electrode system, that facilitates the introduction of a macromolecule, such as a DNA, into cells of a selected tissue in a body or plant. The modular electrode system may comprise a plurality of needle electrodes; a hypodermic needle; an electrical connector that provides a conductive link from a programmable constant-current pulse controller to the plurality of needle electrodes; and a power source. An operator can grasp the plurality of needle electrodes that are mounted on a support structure and firmly insert them into the selected tissue in a body or plant. The macromolecules are then delivered via the hypodermic needle into the selected tissue. The programmable constant-current pulse controller is activated and constant-current electrical pulse is applied to the plurality of needle electrodes. The applied constant-current electrical pulse facilitates the introduction of the macromolecule into the cell between the plurality of electrodes. Cell death due to overheating of cells is minimized by limiting the power dissipation in the tissue by virtue of constant-current pulses. The Cellectra device and system is described in U.S. Patent No. 7,245,963, the contents of which are herein incorporated by reference.

The MID may be an Elgen 1000 system (Inovio Pharmaceuticals). The Elgen 1000 system may comprise device that provides a hollow needle; and fluid delivery means, wherein the apparatus is adapted to actuate the fluid delivery means in use so as to concurrently (for example automatically) inject fluid, the described vaccine herein, into body tissue during insertion of the needle into the said body tissue. The advantage is the ability to inject the fluid gradually while the needle is being inserted leads to a more even distribution of the fluid through the body tissue. It is also believed that the pain experienced during injection is reduced due to the distribution of the volume of fluid being injected over a larger area.

In addition, the automatic injection of fluid facilitates automatic monitoring and registration of an actual dose of fluid injected. This data can be stored by a control unit for documentation purposes if desired.

It will be appreciated that the rate of injection could be either linear or non-linear and that the injection may be carried out after the needles have been inserted through the skin of the subject to be treated and while they are inserted further into the body tissue.

Suitable tissues into which fluid may be injected by the apparatus of the present invention include tumor tissue, skin or liver tissue but may be muscle tissue.

The apparatus further comprises needle insertion means for guiding insertion of the needle into the body tissue. The rate of fluid injection is controlled by the rate of needle insertion. This has the advantage that both the needle insertion and injection of fluid can be controlled such that the rate of insertion can be matched to the rate of injection as desired. It also makes the apparatus easier for a user to operate. If desired means for automatically inserting the needle into body tissue could be provided.

A user could choose when to commence injection of fluid. Ideally however, injection is commenced when the tip of the needle has reached muscle tissue and the apparatus may include means for sensing when the needle has been inserted to a sufficient depth for injection of the fluid to commence. This means that injection of fluid can be prompted to commence automatically when the needle has reached a desired depth (which will normally be the depth at which muscle tissue begins). The depth at which muscle tissue begins could for example be taken to be a preset needle insertion depth such as a value of 4 mm which would be deemed sufficient for the needle to get through the skin layer.

The sensing means may comprise an ultrasound probe. The sensing means may comprise a means for sensing a change in impedance or resistance. In this case, the means may not as such record the depth of the needle in the body tissue but will rather be adapted to sense a change in impedance or resistance as the needle moves from a different type of body tissue into muscle. Either of these alternatives provides a relatively accurate and simple to operate means of sensing that injection may commence. The depth of insertion of the needle can further be recorded if desired and could be used to control injection of fluid such that the volume of fluid to be injected is determined as the depth of needle insertion is being recorded.

The apparatus may further comprise: a base for supporting the needle; and a housing for receiving the base therein, wherein the base is moveable relative to the housing such that the needle is retracted within the housing when the base is in a first rearward position relative to the housing and the needle extends out of the housing when the base is in a second forward position within the housing. This is advantageous for a user as the housing can be lined up on the skin of a patient, and the needles can then be inserted into the patient's skin by moving the housing relative to the base.

As stated above, it is desirable to achieve a controlled rate of fluid injection such that the fluid is evenly distributed over the length of the needle as it is inserted into the skin. The fluid delivery means may comprise piston driving means adapted to inject fluid at a controlled rate. The piston driving means could for example be activated by a servo motor. However, the piston driving means may be actuated by the base being moved in the axial direction relative to the housing. It will be appreciated that alternative means for fluid delivery could be provided. Thus, for example, a closed container which can be squeezed for fluid delivery at a controlled or non-controlled rate could be provided in the place of a syringe and piston system.

The apparatus described above could be used for any type of injection. It is however envisaged to be particularly useful in the field of electroporation and so it may further comprises means for applying a voltage to the needle. This allows the needle to be used not only for injection but also as an electrode during, electroporation. This is particularly advantageous as it means that the electric field is applied to the same area as the injected fluid. There has traditionally been a problem with electroporation in that it is very difficult to accurately align an electrode with previously injected fluid and so user's have tended to inject a larger volume of fluid than is required over a larger area and to apply an electric field over a higher area to attempt to guarantee an overlap between the injected substance and the electric field. Using the present invention, both the volume of fluid injected and the size of electric field applied may be reduced while achieving a good fit between the electric field and the fluid.

The present invention has multiple aspects, illustrated by the following non-limiting examples.

### 4. Examples

### Example 1—Materials and Methods

Mice were housed and treated in a temperature-controlled, light-cycled facility in accordance with the guidelines of the National Institutes of Health (Bethesda, MD, USA) and the University of Pennsylvania (Philadelphia, PA, USA) Institutional Animal Care and Use Committee (IACUC #801577).

Female Dunkin-Hartley guinea pigs weighing between 350 and 450 g and free of intercurrent infection were obtained from Charles River (Wilmington, MA) and were housed at Bio-Quant, Inc., (San Diego, CA) through collaboration with Inovio Pharmaceuticals Inc., PA. The protocol was approved by the Committee on the Ethics of Animal Experiments at the Bio-Quant, Inc. In collaboration with the animal resource dept. of Inovio Pharmaceuticals Inc., (Permit Number: 08-021). At all locations, animals were handled based on the recommendations in the Guide for the Care and Use of Laboratory Animals of the National Institutes of Health. In accordance with the Weatherall report, animal welfare was ensured and steps were taken to ameliorate or minimize.

**Cells and Reagents:** HeLa, 293T and Jurkat (ATCC, Manassas, VA) and HeLa-CD4-TZM-bl (NIH-AIDS Reagent Program, MD) cells were maintained in Dulbecco's modified Eagle's medium (DMEM; Gibco-Invitrogen) supplemented with 10% Fetal Bovine Serum (FBS) and antibiotics and passaged upon confluence. Recombinant HIV-1 envelope gp120 proteins were obtained from Protein Sciences Corporation, Meriden, CT and peroxidise-conjugated streptavidin from Jackson Laboratory. HIV-1 envelope polyclonal antibodies and other viral reagents were obtained through the AIDS Research and Reference Reagent Program, Division of AIDS, NIAID, NIH (Germantown, MD).

**Construction, Expression and Immunization:** The HIV-1 envelope consensus sequences were previously described. Briefly, consensus sequences of HIV-1 envelope from subtype clades A, B, C and D were constructed with modifications as discussed. An IgE leader sequence was added to all envelope antigen sequences to improve expression, and the cytoplasmic tail was truncated to prevent envelope recycling. The resulting optimized HIV-Env DNA immunogens were codon and RNA optimized, and balanced for GCAT content and synthesized, and cloned into the modified pVax1 expression vector. Production of DNA constructs was carried out by Aldevron (Fargo, ND), and purified plasmid DNA was formulated in water for immunization as described.

To test the expression, cells were transfected for expression analysis of synthetic envelopes using the non-liposomal FuGENE transfection reagent (Roche Applied Science, Indianapolis, IN) as suggested by the manufacturer. Briefly, cells were seeded at 70% confluence (50,000 cells per well in 6-well plates) a day before and transfected with 5µg of the HIV-1 envelope plasmids. Cells were harvested 48 to 72 hrs post-transfection in 1 × RIPA buffer (50 mM Tris/HCl (pH 7.4), 150 mM NaCl, 1% Triton X-100, 1% sodium deoxycholate, and 0.1% SDS, supplemented with a complete protease inhibitor cocktail from Roche Applied Science, Indianapolis, IN) and mixed with SDS sample buffer (0.08M Tris (pH 6.8); 2.0% SDS, 10% glycerol, 0.1M dithiothreitol, 0.2% bromophenol blue) before boiling for 5 minutes.

For envelope expression study by immunoblot, specific sera or Abs were diluted 1:100 in PBS and reacted with individual strips for 1h. Subsequently, strips were washed four times with Tris-buffered saline- 0.2% Tween, reacted with a peroxidase-coupled antiserum against mouse IgG (Sigma, St Louis, MO), and incubated with diaminobenzidine substrate (Sigma, St. Louis, MO). For immunofluorescent analysis of optimized envelope expression, RD cells were transfected with vaccine constructs and stained with monoclonal antibodies against HIV-1 envelope (4E10; NIH-AIDS Reagent Program, MD) and against MHC-I prior to confocal analysis. All images were captured by Zeiss 710 LSM Meta microscope system observed in PBS.

**Mice and Guinea Pip Immunizations:** Study 1 was performed in the BALB/c mice, using four groups of animals (4 animals per group/repeated 3 times) which received DNA or protein immunogens alone or in prime-boost combinations as indicated in Fig. 2A. Study 2, for guinea pig study (5 animals/group; repeated 2 times), HIV-1 Env plasmids were immunized as indicated in Figure 6A. Animals in prime-boost and recombinant groups received HIV-1 clade B protein (50µg) formulated with TiterMax adjuvant (Sigma-Aldrich, St Louis, MO) at weeks 8 and 11. A sham immunization group received an empty pVax1 vector and sham protein (PBS) formulated with TiterMax.

At various time points, small amounts of peripheral blood was harvested from the mice and guinea pigs for analysis of the humoral immune response using ELISA assay as indicated in Figure 2A and 6A. For analysis of the cellular immune response, the mice were humanely sacrificed at week 7 and their spleens were used as a source of lymphocytes for the ELISpot and flow cytometry immune analysis. Immunizations were delivered into the quadriceps muscles by in vivo Cellectra^{®} -adaptive EP as described previously. All procedures were performed in accordance with the guidelines of the National Institutes of Health (Bethesda, MD, USA) and the University of Pennsylvania (Philadelphia, PA, USA) Institutional Animal Care and Use Committee.

**T-Cell Elispot Assay:** We determined antigen-specific T-cell responses via IFN-γ ELISpot. Briefly, ELISpot 96-well plates (EMD Millipore Corporation, Billerica, MA) were coated with anti-mouse IFN-γ capture Abs and incubated for 24h at 4°C (R&D Systems, Minneapolis, MN). The following day, plates were washed and blocked for 2h with 1% BSA. Splenocytes from the immunized mice were added to each well and stimulated overnight at 37 °C in 5% CO2 in the presence of RPMI 1640 (negative control), Concanavalin A (positive control), or specific peptide pools (10µg/ml). Peptide pools consist of 15-mer peptides overlapping by 11 amino acids. After 24h of stimulation, the cells were washed and incubated for 24 h at 4°C with biotinylated anti-mouse IFN-γ Abs (R&D Systems, Minneapolis, MN). The plates were washed, and streptavidin—alkaline phosphatase (R&D Systems, Minneapolis, MN) was added to each well and incubated for 2h at room temperature. The plates were then washed and 5-bromo-4-chloro-3'-indolylphosphate p-toluidine salt and nitro blue tetrazolium chloride (chromogen color reagent; R&D Systems, Minneapolis, MN) were added to each well. The plates were then rinsed with distilled water and dried at room temperature. Spots were counted by an automated ELISpot reader (CTL Limited, Shaker Heights, OH).

**B-Cell Elispot Assay:** When testing for antibody-secreting B cells (ASCs), splenocytes were not stimulated prior to detection by ELISpot assay, but instead were tested directly after isolation from the spleen. MultiScreen-IP plates (Millipore, Billerica, MA) were coated with affinity-purified goat anti-mouse IgG (KPL, Gaithersburg, MD) in PBS. Plates were washed six times with PBS and blocked with RPMI with 10% FCS for 2h at room temperature. Splenocytes (10⁵) were added to each well of the ELISpot plate in at least 100µl of medium and incubated overnight at 37°C. Plates were washed six times in PBS with 0.25% Tween 20 (Sigma-Aldrich, St. Louis, MO) (PBS-T) and incubated with 100µl of 1:5,000 biotin-IgG (Jackson ImmunoResearch Laboratories, Inc., West Grove, PA) for 1 h at room temperature. Plates were then washed and incubated with 100µl of 1:60 streptavidin-AP (R&D Research Systems, Minneapolis, MN) for 1h at room temperature. The plates were washed with PBS-T, PBS, and distilled water and developed with 100µl of BCIP/NBT (R&D Research Systems, Minneapolis, MN) for 20 min at room temperature; the reaction was stopped with distilled water. Spots were counted by an automated ELISpot reader (CTL Limited, Shaker Heights, OH). Raw values were determined and multiplied by the appropriate factor so that the data could be represented as ASCs per million splenocytes.

**Intracellular Cytokine Staining:** The phenotype of the responding T cells were analyzed by ICS fluorescence activated cell sorting (FACS) analysis as described. Splenocytes (5 × 10⁶) were stimulated with a 15-mer HIV-1 MN Env peptide pool (2 µg/ml of each peptide; NIH AIDS Research & Reference Reagent Program, MD). Golgi plug (BD Biosciences, San Jose, CA) was added during the final 4 h of incubation. Cells were then stained with anti-mouse CD16/32 (Fc block) antibody, followed by surface staining. Cells were surface stained with CD3-FITC, CD4-Alexa 700, CD8-PerCP (BD Biosciences, San Jose, CA). Cells were then fixed, permeabilized with cytofix cytoperm (BD Biosciences, San Jose, CA) and stained with anti- IFN-γ -PE and anti-IL2-FITC. One million cells per sample were acquired on a BD LSRII flow cytometer (BD Biosciences, San Jose, CA) and CD4⁺ and CD8⁺ events (gated previously on CD3⁺ cells) were gated versus IFN- γ and IL-2. Sample analysis was performed using FlowJo software (Tree Star, Ashland, OR).

**Antibody Binding Assay**: Elisa: For antibody detection, mouse serum samples were collected 7 days after the last immunization. Standard ELISAs were performed using recombinant GP120 as the antigen source, which was prepared as previously described. Antibody binding assays were carried out with either individual animal or pooled sera from the mice or Guinea pig in each group immunized with DNA or DNA+gp120 from different clades of HIV. Briefly, high-binding polystyrene Corning^{®} 96 well plates (Sigma, St Louis, MO) were coated overnight at 4°C with recombinant envelope protein (MN clade B) (2µg/ml) (Protein Sciences Corporation, Meriden, CT), which was diluted in 50 mM carbonate buffer (pH 9.6) and stored overnight at 4°C.

The next day, plates were washed with PBS-T (PBS, 0.05% Tween 20) and blocked for 1h with 3% BSA in PBS-T. Bound IgG was detected using goat anti-mouse IgG-HRP (Sigma, St Louis, MO) at a dilution of 1:5,000. Bound enzyme were detected by the addition of the chromogen substrate solution TMB (R&D Systems, Minneapolis, MN). The enzymatic reaction was stopped with 1N H₂SO₄ and plates were read at a 450-nm wavelength on a Biotek EL312e Bio-Kinetics reader (BioTek, Winooski, VT). All samples were assayed in triplicate. To determine the titers of antibodies after the last immunization, the sera from mice within a group were pooled, serially diluted, and analyzed by ELISA as described above. All samples were assayed in triplicate. End-point titers were calculated as the highest dilution, resulting in a reading of two OD above the va**lue of a negative control serum.**

**HIV-1 Single Round Pseudovirus Production:** The HIV-1 proviral infectious DNA construct pNL4-3/ΔEnv, and primary clade specific envelope isolates were obtained through the AIDS Research and Reference Reagent (RRR)-Program, National Institute of Health (NIH). Pseudoviruses were produced using the pNL4-3/ΔEnv DNA plasmid encoding the HIV backbone and a plasmid encoding multiple primary as well as laboratory viruses, including clade A (SF162 LS; NL4-3 and 92RW020) clade B (MN/H9; Bal.26; 6535.3 and HXB2); clade C (MW965.26); and clade A/G (DJ263) envelope variants. HIV-1 pseudoviral particles were generated by transfection with pNL4-3/ΔEnv alone or co-transfection with a panel of envelope plasmids by FuGENE 6 transfection in 293T cells. Two to three days after transfection, virion-containing culture supernatants were harvested, precleared by centrifugation at 1,200 rpm for 7 min and filtered through a 0.45-µm pore-size membrane. Cleared culture supernatants were then treated with DNase I (Roche Applied Science, Indianapolis, IN) at a final concentration of 20µg/ml at 37°C for 1 h and aliquots in 300-µl fractions were saved at -80°C until needed. The p24 concentrations of the virus stocks were quantified by HIV-1 p24 antigen ELISA as described. Titration of pseudotyped virus was determined using the 50% tissue culture infectious dose (TC-ID₅₀) assay in TZM-BI cells.

**HIV-1 Neutralization Assay:** Neutralization titers were measured as a function of the reduction in luciferase reporter gene expression after a single round of viral infection in TZM-B1 cells as previously described [40]. TZM-B1 cells were obtained through the NIH AIDS Research and Reference Reagent Program. These cells are engineered to express CD4 and CCR5 and contain integrated reporter genes for firefly luciferase and E. coli β-galactosidase under control of an HIV-1 LTR.

Guinea pig sera were heat-inactivated at 56 °C for 1h prior to the assay. 25µl of sera from the four groups were diluted in 125µl of PBS. The diluted sera were further diluted threefold in a 96-well plate. Fifty microliters of a cell-free virus (200 TCID₅₀) were added to each well. After 1h of incubation, a 10,000 TZM-B1 cell suspension was added to each well. The plates were incubated for 48h, after which 20µl of lysis buffer (Cell Culture Lysis Reagent, Promega. Madison, WI) was added to each well at room temperature for 10min and followed by 100µl of Bright-Glo substrate and buffer (Luciferase Assay system, Promega, Madison, WI). The plate was read immediately with Glomax Luminometer (Promega, Madison, WI). The percentages of RLU reduction were calculated as (1—(average RLU of duplicates with sample sera―control wells)/(average RLU from mock control sera―control wells)) × 100%. Neutralizing antibody titers were expressed as the reciprocal of the serum dilution required to reduce the RLU by 50%.

**Statistical Analysis**: Group analyses were completed by matched, two-tailed, unpaired t-test and all values are mean ± SEM. Statistical analyses were performed by GraphPad Prism Software (La Jolla, CA). Statistically significant differences between groups were defined as ^{∗}P < 0.1, ^{∗∗}P < 0.01, ^{∗∗∗}P < 0.001, and ^{∗∗∗∗}P < 0.0001.

### Example 2: Designs of HIV-1 Envelope Consensus DNA Vaccines

HIV-1-Envelope was constructed. Several modifications were made in constructing the plasmid including addition of a highly efficient IgE leader peptide sequence to facilitate expression. The resulting Env plasmid expressed at high levels in tissue culture as shown by FACS analysis in Jurkat cells (Figure 1A). To verify the expression of the consensus HIV-1 Env plasmids, an indirect immuno fluorescence assay (IFA) with confocal microscopy of transfected RD cells was performed, using a anti-HIV-1 Env 4E10 mAbs antibody, we observed colocalization of the specific cell membrane expression of Env with MHC-1 expression (Figure 1B). No expression was detected in control vector (pVax1) transfected cells.

### Example 3: Improved CD8 T-Cell Responses by Optimized HIV-1 Envelope Plasmid

Vaccine immunogenicity was assessed in vivo following immunization performed in mice. Mice were immunized with 25ug each Env plasmid DNA by using adaptive EP at week 0 and 2 (Figure 2A). Mice receiving the protein boost were then immunized intramuscularly with 50µg of HIV-1 MN gp120 formulated Titermix X-Gold adjuvant at weeks four and six. One week post final immunization, mice were sacrificed and splenocytes were isolated to test the T-cell responses using an ELISpot assay, using the corresponding envelope peptide pools for stimulation. An increased number of Env-specific IFN-γ producing cells were observed in vaccinated animals, whereas control (pVax1) group splenocytes showed no response to Env peptide (Figure 2B). While DNA vaccination alone was superior to recombinant protein alone, the combination induced the best response in all cases. Interestingly, clade A, C and D group, DNA primes were boosted by a mismatched clade B antigen. Furthermore, mice immunized with the combined multi-clade DNA vaccine developed considerably higher IFN-γ production by CD8 T cells than mice injected with single-clade vaccines. There did not appear to be any antigen competition between envelopes (Figure 2C).

### Example 4: Antigen Specific T- Cells Produce IFN-δ and IL-2 After DNA Prime-Protein Boost Immunization

Given the importance of polyfunctional T cell responses in controlling HIV-1 infection, we measured the ability of multiclade-specific T cell populations from immunized mice to secrete IFN-γ and IL-2 in response to envelope peptide pool stimulation. Functionally divergent Ag-specific T-cell populations are commonly defined by secretion of IL-2 by CD4 T cells populations and IFN-γ by CD8 T cell populations. Therefore, we characterized Ag-specific CD8⁺ and CD4⁺ T-cell responses by simultaneous measurement of both IFN-γ and IL-2 secretion following peptide stimulation as described in Materials and Methods. We further analyzed the phenotype of the adaptive immune response elicited in DNA+protein immunization groups by polychromatic flow cytometry, using FACS- based ICS, we evaluated IFN-γ and IL-2 after in vitro stimulation with HIV-1 Env peptide pools that covered the entire Env sequence. Our gating strategy for intracellular cytokine flow cytometric analysis is depicted in Figure 3A; this strategy allowed us to separate CD8⁺ or CD4⁺ T cells into subsets based on their ability to produce one or more cytokines. As shown in Figure 3B a multi-clade DNA prime and protein boost induced higher levels of IFN-γ/IL-2 secretion in CD8⁺/CD4⁺ T-cells (Figure 3B & C). We observed that the percentage of Env-specific IFN-γ⁺ -secreting CD8⁺ Tcells was greater as that of IL-2⁺ secreting CD4⁺ T-cells, suggesting the induction of a more dominant CD8⁺ T-cell response observed in multi clade vaccine. Another effect of the DNA prime-protein boost strategy was an increase in the CD8⁺ /IFN-γ⁺ and CD4⁺/IL-2⁺ T-cell populations as compared to the CD8⁺ /IL-2⁺ and CD4⁺/IFN_{-γ}⁺ populations. Consistent with the results observed in the IFN-γ ELISpot, the combined DNA prime-protein boost generated more robust Env specific T-cell responses. This result supports the conclusion that optimized DNA, in combination with EP delivery and vaccination with a protein boost, is able to induce expansion of functional Env-specific CD8⁺ and CD4⁺ T-cells.

### Example 5: Effects of Multiple Clade Formulations and Protein Boost Regimens on ABS Elicitation and B-Cell Activation

Next, we wanted to test whether immunization with multiple Env variants induced higher titers of antibodies than immunization with any single construct alone. We measured the levels of Env-specific IgG production using pooled serum from the groups of mice by binding ELISA to HIV-1 Env. Surprisingly, DNA alone elicited higher antibody binding titers than single recombinant protein alone. However, a DNA prime-protein boost, induced more robust binding titers than either DNA or protein alone (Figure 4). More importantly, the multiple-clade immunization elicited higher titers than did immunization with any single Env variant alone (Figure 4B). Titers from the multiple-clade immunization were further enhanced with the addition of a protein boost. These studies illustrate that an improved humoral response is generated with a multi-clade DNA prime followed by recombinant protein boost.

To further understand the mechanisms underlying antibody production, B-cell ELISpots were performed to measure the frequency of vaccine-induced antibody secreting cells (ASCs). As indicated in Figure 5A and Figure 5B, we observed higher levels of HIV-1-specific IgG producing B-cells following a DNA prime than protein boost. Also these results demonstrate that a DNA vaccine combined with EP can activate a robust humoral response as measured by Env-specific IgG production by B-cell ELIspot. The B-cell response was enhanced by approximately 50% with the inclusion of a recombinant protein boost. Taken together, these data support the conclusion that optimized DNA alone can induce a strong humoral response and the effect is further enhanced by the addition of a protein boost. Further, we also observed a positive correlation between antibody titer to B-cell ELIspot in the multi-clade DNA prime followed by a recombinant protein boost immunization strategies (Figure 5C).

### Example 6-Antibody Levels in Guinea Pigs After DNA Prime Protein Boost Immunization

We further characterized the humoral response induced by the DNA prime-protein boost regimen in guinea pigs using three 25µg DNA immunizations with EP followed by two 50 µg protein boosts (Figure 6A). Guinea pigs allow for additional investigation of antibody responses since neutralization assays using TZM-B1 cells can be easily performed without the isolation of IgG as would be necessary in mouse serum. As seen in mice, the DNA prime-protein boost immunized guinea pigs exhibited the highest level of antibody responses as judged by binding ELISA. Similarly consistent with our mouse studies was the fact that multi-clade DNA alone resulted in superior levels of binding antibodies as compared to recombinant protein alone (Figure 6B&C).

To further characterize Env-specific responses induced in immunized guinea pigs, sera from DNA prime and protein boost animals were tested for seroconversion using Western blot analysis to confirm specificity. Protein lysates were isolated from HIV-1 clade Env transfected 293T cells and resolved using SDS-PAGE. Figure 6D shows that sera from multi clade Env-immunized guinea pigs bound to Env protein. Consistent with antibody analysis by ELISA, guinea pigs vaccinated with a multi-clade DNA vaccine exhibited a high level of cross reactivity with envelope proteins from different clades and demonstrate that the multi-clade DNA prime and protein boost vaccination procedure provided a technical platform to allow us to develop more effective HIV vaccines by using polyvalent Env formulations.

### Example 7-Neutralizing Antibody Response in Guinea Pigs

Sera from immunized guinea pigs were analyzed for the level and specificity of NAbs activity to define antibody functionality induced by a multi-clade DNA prime-protein boost. Guinea pigs sera were used because serum IgG does not need to be isolated as in mice for neutralization studies. The serum neutralization titer was determined by assessing whether sera could neutralize 50% of the virus infection using the TZM-bl cell assay system. Sera were tested at in triplicate (1:50 dilution) to test specificity.

We observed that immunization with a combination of Env clades A, B, C and D primed and protein boost was capable of generating NAbs using a standardized tier1a and tier 1b panel of reference Env pseudoviruses for Nab assessment (Figure 7). Out of the nine viruses from different clades that were tested, six viruses were neutralized with pooled sera from the multi-clade DNA vaccination and protein boost group. In contrast, vaccination with recombinant protein alone exhibited very limited neutralization breadth: sera from this group showed detectable neutralization titers for only few viruses out of the nine viruses tested. Recombinant protein vaccination also showed limited cross-clade breadth.

Similar to the results from binding titer assays, the magnitude of Nabs production was enhanced by a DNA prime-protein boost vaccination protocol. The addition of a recombinant protein boost enhanced the titers generated by DNA alone by half a log for many of the viruses. These data suggest that a multi-clade DNA prime is capable of generating improved neutralization breadth with lower titer and the magnitude of this response is enhanced by the addition of a recombinant protein boost without decreasing breadth. Overall, the DNA prime-protein boost regimen yielded superior results in all antibody assays.

### Example 8: Discussion

The major limitation of DNA vaccination in the past has been its relatively weak immunogenicity in vivo. Recent DNA studies using in vivo EP have suggested that this new generation of DNA vaccine is more immune potent and this result for cellular response has been observed in humans. Prior studies by our lab and others exploring the use of first generation HIV DNA vaccines showed that weak binding antibodies to Env could be elicited through DNA technology mostly in small animals. Antibody responses were enhanced by boosting with monomeric gp120 protein, but the NAbs were of low potency and exhibited little cross-reactivity. We sought to improve upon these results by introducing changes to our vaccine platform that would redirect the immune system towards a stronger humoral response. These changes included enhanced consensus DNA vaccines with the potential to induce antigen expression on the cell surface, improvements in transfection efficiency and antigen presentation via adaptive electroporation, and the addition of a recombinant protein boost to further stimulate B-cell expansion.

The first DNA vaccines produced in the early 1990s were not able to induce reasonable levels of cell mediated immunity (CMI) in either nonhuman primates or humans. Through recent advances in DNA construct design and delivery methods, this approach now can produce strong CD8+ T-cell responses in these species and most importantly in two human clinical trails for HPV and HIV strong T cell response were included in human by synthetic consensus optimized DNA delivered by adaptive EP. We follow upon this encourage data in this prime boost studies presented here. We repeat that that vaccination with DNA plasmids encoding synthetic consensus envelope immunogens induce T-cell responses which are superior to protein alone. We also demonstrate that the most robust responses were achieved with a combined DNA prime-protein boost strategy, which particularly enhanced CD4 responses. When investigating the effects of a multi-clade DNA prime, we found that a combination of clades A, B, C and D improved T-cell responses as determined by both ELISpot and flow cytometry analyses. We considered the possibility that this phenomenon was the result of a dose effect since the total amount of DNA administrated to the multi-clade was 100µg (25µg of each clade) in comparison to 25µg for the single-clade group. In order to control for this variable, we conducted a dosing study in which mice were given a range of doses for one single-clade vaccine. It was determined that doses >25µg DNA did not significantly improve the immune response and therefore dosing alone could not explain the observed improvement (data not shown). A more likely explanation for this phenomenon is that a greater number of T-cells are activated from a multi-clade vaccine due to a greater number of unique epitopes presented by the vaccine. Interestingly, a multi-clade DNA prime-protein boost also appeared to drive a more functionally divergent T-cell response. Protein and DNA only vaccination produced roughly similar numbers of IFN-γ+/CD8+, and IFN-γ+/CD4+ T-cells, as well as similar numbers of IL-2+/CD4+ and IL-2+/CD8+ T- cells. In contrast, a multi-clade DNA prime-protein boost resulted in four times more IFN-γ+-secreting CD8+ T-cells than IFN-γ+-secreting CD4+ T-cells and higher IL-2+ -secreting CD4+ and CD8+ T-cells. Further studies will be required to elucidate the exact mechanistic and phenotypic T-cell differences generated from the enhanced DNA prime-protein boost platform.

In addition to the induction of T-cell responses, consensus envelope vaccination also induced a robust humoral response. We observed that a multi-clade DNA vaccine induced higher antibody titers than the recombinant protein vaccine alone. Such results were surprising considering that recombinant protein has traditionally been the gold standard for antibody-based vaccines. These results may be due to the immunization with the recombinant protein used in this study. A different protein and adjuvant system may also have resulted in higher titers more consistent with those observed from our DNA vaccines. Despite the low antibody activity achieved with a single immunization of rgp120 alone, it served as an effective boosting agent, enhancing binding titers following a single or multi-clade DNA prime. Between the single- and multi-clade DNA primes, greater antibody levels were achieved with the multi-clade formulation. Similar to the T-cell responses, this might be a consequence of greater stimulation by a larger number of unique epitopes.\

Another important feature of a future HIV vaccination is the breadth of the humoral response. Vaccination with clade A or clade C- DNA immunogens produced binding titers for clade B gp120 equal to those produced by a clade B DNA vaccine. We have similarly observed broad cross-reactive responses with some of our other synthetic consensus vaccines. These broad responses are most likely attributable to the consensus design in which only the most common amino acid sequences are expressed, resulting in the presentation of conserved epitopes. The DNA platform itself may also contribute to the observed breadth, since the final gp140 protein product is more likely to be expressed in a functionally relevant trimeric form with natural glycosylation patterns. We are currently investigating the biological properties of consensus gp140 produced in host cells, focusing particularly on the oligomeric state and localization of the final protein product.

Similar to the data observed for antibody production in mice, the consensus DNA vaccine induced broader neutralizing titers when administered in guinea pigs. A consensus clade A, B, C and D vaccine induced titers against several primary clade viruses. In contrast, clade B recombinant protein induced only clade B neutralizing antibodies. Despite only inducing clade B NAbs when administered alone, recombinant protein delivered after a DNA prime was able to significantly improve the induction of clade A, B, C and D NAbs titers.

Our data support that enhanced DNA vaccines delivered by EP are useful for the induction of strong binding antibody responses against a broad range of viral strains. Combining DNA with a protein boost elicited enhanced NAbs activity against a panel of viral isolates. Thus, the data provide important support that enhanced adaptive EP delivered DNA prime-protein boost is an important strategy for delivering polyvalent Env-based HIV vaccines aimed at improving breath. Further immunization exploration of the immune response induced by diverse HIV DNA cassettes in combination with multiple protein boosts will likely provide important information of relevance to HIV vaccine development.

### 5. Clauses

Clause 1. A composition comprising (a) a first vaccine, wherein the first vaccine comprises at least one, at least two, at least three, or at least four nucleic acids, wherein each nucleic acid encodes an antigen, and wherein the at least one, at least two, at least three, or at least four nucleic acids are selected from the group consisting of a nucleic acid encoding a HIV-1 subtype A consensus antigen, a nucleic acid encoding a HIV-1 subtype B consensus antigen, a nucleic acid encoding a HIV-1 subtype C consensus antigen, a nucleic acid encoding a HIV-1 subtype D consensus antigen, and any combination thereof; and (b) a second vaccine, wherein the second vaccine comprises at least one, at least two, at least three, or at least four antigenic peptides, and wherein the at least one, at least two, at least three, or at least four antigenic peptides are selected from the group consisting of a HIV-1 subtype A consensus peptide, a HIV-1 subtype B consensus peptide, a HIV-1 subtype C consensus peptide, a HIV-1 subtype D consensus peptide, and any combination thereof.

Clause 2. The composition of clause 1, wherein the at least two nucleic acids of the first vaccine comprises (a) a first nucleic acid encoding a HIV-1 subtype A consensus antigen, a HIV-1 subtype B consensus antigen, a HIV-1 subtype C consensus antigen, or a HIV-1 subtype D consensus antigen, and (b) a second nucleic acid encoding a HIV-1 subtype A consensus antigen, a HIV-1 subtype B consensus antigen, a HIV-1 subtype C consensus antigen, or a HIV-1 subtype D consensus antigen.

Clause 3. The composition of clause 1, wherein the at least three nucleic acids of the first vaccine comprises: (a) a first nucleic acid encoding a HIV-1 subtype A consensus antigen, a HIV-1 subtype B consensus antigen, a HIV-1 subtype C consensus antigen, or a HIV-1 subtype D consensus antigen, (b) a second nucleic acid encoding a HIV-1 subtype A consensus antigen, a HIV-1 subtype B consensus antigen, a HIV-1 subtype C consensus antigen, or a HIV-1 subtype D consensus antigen; and (c) a third nucleic acid encoding a HIV-1 subtype A consensus antigen, a HIV-1 subtype B consensus antigen, a HIV-1 subtype C consensus antigen, or a HIV-1 subtype D consensus antigen.

Clause 4. The composition of clause 1, wherein the at least four nucleic acids of the first vaccine comprises (a) a first nucleic acid encoding a HIV-1 subtype A consensus antigen, a HIV-1 subtype B consensus antigen, a HIV-1 subtype C consensus antigen, or a HIV-1 subtype D consensus antigen; (b) a second nucleic acid encoding encoding a HIV-1 subtype A consensus antigen, HIV-1 subtype B consensus antigen, a HIV-1 subtype C consensus antigen, or a HIV-1 subtype D consensus antigen; (c) a third nucleic acid encoding a HIV-1 subtype A consensus antigen, a HIV-1 subtype B consensus antigen, a HIV-1 subtype C consensus antigen, or a HIV-1 subtype D consensus antigen; and (d) a fourth nucleic acid encoding a HIV-1 subtype A consensus antigen, a HIV-1 subtype B consensus antigen, a HIV-1 subtype C consensus antigen, or a HIV-1 subtype D consensus antigen.

Clause 5. The composition of clause 1, wherein the antigen of the first vaccine is selected from the group consisting of Env A, Env B, Env C, Env D, B Nef-Rev, Gag, gp120 and gp140.

Clause 6. The composition of clause 1, wherein the antigen of the second vaccine is selected from the group consisting of Env A, Env B, Env C, Env D, B Nef-Rev, Gag, gp120 and gp140.

Clause 7. The composition of clause 1, wherein the antigenic peptide of the first vaccine is gp140.

Clause 8. The composition of clause 1, wherein the antigenic peptide of the second vaccine is gp120.

Clause 9. The composition of clause 1, wherein the first vaccine is a priming vaccine.

Clause 10. The composition of clause 1, wherein the second vaccine is a boosting vaccine.

Clause 11. A method of immunizing a subject in need thereof against HIV-1, the method comprising administering the composition of clause 1 to the subject, wherein the first vaccine is administered independently of the second vaccine.

Clause 12. The method of clause 11, wherein first vaccine is administered at a first time, and wherein the first time is day 1 of a vaccination regimen.

Clause 13. The method of clause 12, wherein the first vaccine is administered at a second time, and wherein the second time is within 12 hours, 24 hours, 36 hours, or 48 hours of the first time of the first vaccine.

Clause 14. The method of clause 13, wherein the first vaccine is a priming vaccine.

Clause 15. The method of clause 11, wherein the second vaccine is administered about 48 hours to about 15 weeks, about 48 hours to about 10 weeks, about 48 hours to about 5 weeks, or about 48 hours to about 1 week after the first vaccine.

Clause 16. The method of clause 11, wherein the second vaccine is administered at least about 48 hours, at least about 90 hours, at least about 2 weeks, at least about 5 weeks, or at least about 10 weeks after the first vaccine.

Clause 17. The method of clause 11, wherein the second vaccine is administered a second time, and wherein the second time is about 48 hours to about 15 weeks, about 48 hours to about 10 weeks, about 48 hours to about 5 weeks, or about 48 hours to about 1 week after the first administration of the second vaccine.

Clause 18. The method of clause 11, wherein the second vaccine is administered a second time, and wherein the second time is at least about 48 hours, at least about 90 hours, at least about 2 weeks, at least about 5 weeks, or at least about 10 weeks after the first administration of the second vaccine.

Clause 19. The method of clause 11, wherein the first vaccine is administered 1 time, 2 times, 3 times, 4 times, or 5 times, each administration of the first vaccine being spaced in time from the other administrations of the first vaccine.

Clause 20. The method of clause 9, wherein the second vaccine is administered 1 time, 2 times, 3 times, 4 times, or 5 times, each administration of the second vaccine being spaced in time from the other administrations of the second vaccine.

Further embodiments of the present invention are provided below:
1. A composition comprising
   (a) a first vaccine, wherein the first vaccine comprises at least one, at least two, at least three, or at least four nucleic acids, wherein each nucleic acid encodes an antigen, and wherein the at least one, at least two, at least three, or at least four nucleic acids are selected from the group consisting of a nucleic acid encoding a HIV-1 subtype A consensus antigen, a nucleic acid encoding a HIV-1 subtype B consensus antigen, a nucleic acid encoding a HIV-1 subtype C consensus antigen, a nucleic acid encoding a HIV-1 subtype D consensus antigen, and any combination thereof; and
   (b) a second vaccine, wherein the second vaccine comprises at least one, at least two, at least three, or at least four antigenic peptides, and wherein the at least one, at least two, at least three, or at least four antigenic peptides are selected from the group consisting of a HIV-1 subtype A consensus peptide, a HIV-1 subtype B consensus peptide, a HIV-1 subtype C consensus peptide, a HIV-1 subtype D consensus peptide, and any combination thereof.
2. The composition of embodiment 1, wherein the at least two nucleic acids of the first vaccine comprises
   (a) a first nucleic acid encoding a HIV-1 subtype A consensus antigen, a HIV-1 subtype B consensus antigen, a HIV-1 subtype C consensus antigen, or a HIV-1 subtype D consensus antigen, and
   (b) a second nucleic acid encoding a HIV-1 subtype A consensus antigen, a HIV-1 subtype B consensus antigen, a HIV-1 subtype C consensus antigen, or a HIV-1 subtype D consensus antigen.
3. The composition of embodiment 1, wherein the at least three nucleic acids of the first vaccine comprises:
   (a) a first nucleic acid encoding a HIV-1 subtype A consensus antigen, a HIV-1 subtype B consensus antigen, a HIV-1 subtype C consensus antigen, or a HIV-1 subtype D consensus antigen,
   (b) a second nucleic acid encoding a HIV-1 subtype A consensus antigen, a HIV-1 subtype B consensus antigen, a HIV-1 subtype C consensus antigen, or a HIV-1 subtype D consensus antigen; and
   (c) a third nucleic acid encoding a HIV-1 subtype A consensus antigen, a HIV-1 subtype B consensus antigen, a HIV-1 subtype C consensus antigen, or a HIV-1 subtype D consensus antigen.
4. The composition of embodiment 1, wherein the at least four nucleic acids of the first vaccine comprises
   (a) a first nucleic acid encoding a HIV-1 subtype A consensus antigen, a HIV-1 subtype B consensus antigen, a HIV-1 subtype C consensus antigen, or a HIV-1 subtype D consensus antigen;
   (b) a second nucleic acid encoding encoding a HIV-1 subtype A consensus antigen, HIV-1 subtype B consensus antigen, a HIV-1 subtype C consensus antigen, or a HIV-1 subtype D consensus antigen;
   (c) a third nucleic acid encoding a HIV-1 subtype A consensus antigen, a HIV-1 subtype B consensus antigen, a HIV-1 subtype C consensus antigen, or a HIV-1 subtype D consensus antigen; and
   (d) a fourth nucleic acid encoding a HIV-1 subtype A consensus antigen, a HIV-1 subtype B consensus antigen, a HIV-1 subtype C consensus antigen, or a HIV-1 subtype D consensus antigen.
5. The composition of embodiment 1, wherein the antigen of the first vaccine is selected from the group consisting of Env A, Env B, Env C, Env D, B Nef-Rev, Gag, gp120 and gp140.
6. The composition of embodiment 1, wherein the antigen of the second vaccine is selected from the group consisting of Env A, Env B, Env C, Env D, B Nef-Rev, Gag, gp120 and gp140.
7. The composition of embodiment 1, wherein the antigenic peptide of the first vaccine is gp140.
8. The composition of embodiment1, wherein the antigenic peptide of the second vaccine is gp120.
9. The composition of embodiment 1, wherein the first vaccine is a priming vaccine.
10. The composition of embodiment 1, wherein the second vaccine is a boosting vaccine.
11. A method of immunizing a subject in need thereof against HIV-1, the method comprising administering the composition of embodiment 1 to the subject, wherein the first vaccine is administered independently of the second vaccine.
12. The method of embodiment 11, wherein first vaccine is administered at a first time, and wherein the first time is day 1 of a vaccination regimen.
13. The method of embodiment 12, wherein the first vaccine is administered at a second time, and wherein the second time is within 12 hours, 24 hours, 36 hours, or 48 hours of the first time of the first vaccine.
14. The method of embodiment 13, wherein the first vaccine is a priming vaccine.
15. The method of embodiment 11, wherein the second vaccine is administered about 48 hours to about 15 weeks, about 48 hours to about 10 weeks, about 48 hours to about 5 weeks, or about 48 hours to about 1 week after the first vaccine.
16. The method of embodiment 11, wherein the second vaccine is administered at least about 48 hours, at least about 90 hours, at least about 2 weeks, at least about 5 weeks, or at least about 10 weeks after the first vaccine.
17. The method of embodiment 11, wherein the second vaccine is administered a second time, and wherein the second time is about 48 hours to about 15 weeks, about 48 hours to about 10 weeks, about 48 hours to about 5 weeks, or about 48 hours to about 1 week after the first administration of the second vaccine.
18. The method of embodiment 11, wherein the second vaccine is administered a second time, and wherein the second time is at least about 48 hours, at least about 90 hours, at least about 2 weeks, at least about 5 weeks, or at least about 10 weeks after the first administration of the second vaccine.
19. The method of embodiment 11, wherein the first vaccine is administered 1 time, 2 times, 3 times, 4 times, or 5 times, each administration of the first vaccine being spaced in time from the other administrations of the first vaccine.
20. The method of embodiment 9, wherein the second vaccine is administered 1 time, 2 times, 3 times, 4 times, or 5 times, each administration of the second vaccine being spaced in time from the other administrations of the second vaccine.

## Claims

1. A combination prime-boost vaccine comprising
(a) a prime vaccine, wherein the prime vaccine comprises a multi-clade vaccine comprising a nucleic acid molecule encoding a HIV-1 subtype A peptide, a nucleic acid molecule encoding a HIV-1 subtype B peptide, a nucleic acid molecule encoding a HIV-1 subtype C peptide and a nucleic acid molecule encoding a HIV-1 subtype D peptide; and
(b) a boosting vaccine, wherein the boosting vaccine comprises a HIV-1 subtype B peptide;
for use in a method of generating a therapeutically effective immune response against HIV-1 in a subject in need thereof, wherein the priming vaccine is administered independently of the boosting vaccine and wherein the boosting vaccine increases the immune response to the priming vaccine.

2. The combination prime-boost vaccine for use according to claim 1, wherein the HIV-1 subtype A peptide of the priming vaccine is Env A, wherein the HIV-1 subtype B peptide of the priming vaccine is Env B, wherein the HIV-1 subtype C peptide of the priming vaccine is Env C, and wherein the HIV-1 subtype D peptide of the priming vaccine is Env D.

3. The combination prime-boost vaccine for use according to claim 1, wherein the HIV-1 subtype B peptide of the boosting vaccine is gp120.

4. The combination prime-boost vaccine for use according to any one of claims 1 to 3, wherein the priming vaccine is administered at a first time, and wherein the first time is day 1 of a vaccination regimen, optionally wherein the priming vaccine is administered at a second time, and wherein the second time is within 12 hours, 24 hours, 36 hours, or 48 hours of the first time of the priming vaccine.

5. The combination prime-boost vaccine for use according to any one of claims 1 to 4, wherein the boosting vaccine is administered about 48 hours to about 15 weeks, about 48 hours to about 10 weeks, about 48 hours to about 5 weeks, about 48 hours to about 1 week, at least about 48 hours, at least about 90 hours, at least about 2 weeks, at least about 5 weeks, or at least about 10 weeks after the priming vaccine.

6. The combination prime-boost vaccine for use according to any one of claims 1 to 5, wherein the boosting vaccine is administered a second time, and wherein the second time is about 48 hours to about 15 weeks, about 48 hours to about 10 weeks, about 48 hours to about 5 weeks, or about 48 hours to about 1 week, at least about 48 hours, at least about 90 hours, at least about 2 weeks, at least about 5 weeks, or at least about 10 weeks after the first administration of the boosting vaccine.

7. The combination prime-boost vaccine for use according to any one of claims 1 to 6, wherein the priming vaccine is administered 1 time, 2 times, 3 times, 4 times, or 5 times, each administration of the priming vaccine being spaced in time from the other administrations of the priming vaccine, or wherein the boosting vaccine is administered 1 time, 2 times, 3 times, 4 times, or 5 times, each administration of the boosting vaccine being spaced in time from the other administrations of the boosting vaccine.
